# EUROPEAN PATENT APPLICATION

(11) **EP 2 479 183 A2**
(43) Date of publication of application: **25.07.2012**
(21) Application number: 11155897.9
(22) Date of filing: 04.11.2005
(51) Int. Cl.: C07J 71/00, A61K 31/58, A61P 3/04, A61P 25/28

(54) **Polymorphs of sarsasapogenin**

(30) Priority: 05.11.2004 GB 0424528
(62) Divisional of application: 05799915.3
(71) Applicant: Phytopharm PLC, Ermine Business Park Huntingdon Cambridgeshire PE29 6UA (GB)
(72) Inventor: Tiffin, Peter David, Huntingdon, Cambridgeshire PE29 6UA (GB)
(74) Representative: Barnes, Colin Lloyd

(57) **Abstract**

The invention provides sarsasapogenin in novel amorphous, crystalline, solvated and hydrated forms, and the use thereof in manufacturing pharmaceutical or edible grade sarsasapogenin and its derivatives

## Description

### Field of the Invention

The present invention relates to novel amorphous and crystalline forms of sarsasapogenin and its hydrates and solvates.

### Background to the Invention

It is well established that some organic compounds can crystallise in a number of different polymorphic forms or crystal habits, which may comprise the compound as such, solvates of the compound, hydrates of the compound, or combinations thereof. Alternatively, the compound, solvate or hydrate may precipitate as an amorphous solid.

The stability and bioavailability of the drug product may vary according to the polymorphic form present. The choice of crystal form is thus a critical aspect of drug development (Brittain, Pharm. Tech. pp. 50-52, 1994; Yu et al., Pharm. Sci. Techno/. Today, 1, pp. 118 to 127, 1998; Byrn et al., Chem. Mater. 6, pp. 1148 to 1158, 1994; Byrn et al., Pharm. Res. , 12, pp. 945 to 954, 1995; Henk et al., Pharm. Ind. 59, pp. 165 to 169, 1997).

Sarsasapogenin is an A/B-*cis* spirostane steroidal sapogenin having the structure:

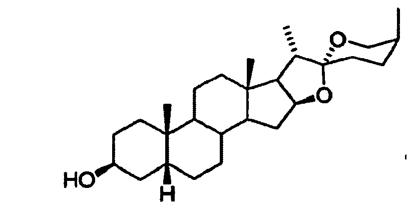

Derivatives are known, of which there may particularly be mentioned esters formed with the free OH group at the 3-position carbon atom in the A (left hand end) ring, for example carboxylic acid esters such as sarsasapogenin acetate.

Sarsasapogenin and its derivatives have been identified as valuable therapeutic agents in human and veterinary medicine and in non-therapeutic human and non-human animal treatments. See, for example, US Patent No. 4680289 (use of sarsasapogenin against obesity and diabetes obesity syndromes); Yi et al, Synthesis and Applications of Isotopically Labelled Compounds, 315 to 320, 1997 (Ed. J R Heys and D G Melillo) (use of sarsasapogenin against senile dementia); WO-A-99/48507 (use of sarsasapogenin against conditions characterised by a deficiency in membrane-bound receptor number or function); WO-A-01/23406 and WO-A-01/49703 (use of sarsasapogenin derivatives against cognitive dysfunction and allied conditions, including non-therapeutic use to enhance cognitive function in mentally healthy humans and animals); and WO-A-02/079221 and WO-A-03/082893 (use of sarsasapogenin and derivatives thereof against non-cognitive neurodegeneration, non-cognitive neuromuscular degeneration, motor-sensory neurodegeneration and loss of receptor function in the absence of cognitive, neural or neuromuscular impairment).

In addition, sarsasapogenin is known as an important precursor in the manufacture of other steroidal compounds.

It has been reported that recrystallisation of sarsasapogenin from acetone gave large prismatic crystals with a melting point of 199-199.5°C (Simpson and Jacobs, J. Biol. Chem., 105, 501 to 510, 1934).

Recrystallisation of sarsasapogenin from acetone gave a product with a melting point of 199.5-200°C, which was unchanged on crystallisation from alcohol (Simpson and Jacobs, J. Biol. Chem., 109, 573 to 584, 1935). These authors reported that the melting point of the product obtained upon recrystallisation from ethyl acetate possessed a melting point of 194-195°C and attributed this to polymorphism since elemental analytical data was consistent with the molecular formula C₂₇H₄₄O₃.

In J. Am. Chem. Soc. pp. 846 to 851, 1939, Marker *et al.* reported a melting point of 200°C for sarsasapogenin.

Marker et al. (J. Am. Chem. Soc. 65, pp. 1199 to 1209, 1943, at p. 1207) reported that sarsasapogenin acetate shows polymorphic forms melting at 126-129 and 138 to 142 °C. The melting point of sarsasapogenin from a number of sources was always in the range 199-202°C. However, the recrystallisation solvent was not stated and the article made no mention of polymorphic forms of sarsasapogenin.

In J. Am. Chem. Soc. 77, pp. 5661 to 5665, 1955 Wall *et al.* reported that sarsasapogenin crystallised as plates from acetone with a melting point of 200°C.

Scheer *et al.* crystallised sarsasapogenin ethyl acetate from light petroleum and observed a melting point of 198-199°C (Scheer et al., J. Am. Chem. Soc., 77, pp. 641 to 646,1955).

Wall et al., (J. Biol. Chem., 198, pp. 533 to 543, 1952) reported the melting point of sarsasapogenin to be 200°C. However, the recrystallisation solvent was not stated. The paper reported that the use of a Kofler microscopic melting point apparatus having polarizing disks allowed for the crystal form or habit to be observed. No mentioned was made of polymorphism but the impact of impurities upon the melting point was noted.

Parsons et al. (Henry Ford Hosp. Med. Bull., 12, pp. 87 to 120, 1964) described a specific crystalline form of sarsasapogenin by X-ray powder diffraction (XRPD). From the data presented it cannot be concluded that this form corresponds to any of the forms described herein.

The prior art publications acknowledged above are incorporated herein by reference.

Depending on the administration route desired in the therapy, it may be desirable to improve or at least control the stability and water solubility of the sarsasapogenin, to obtain a desired bioavailability profile. Furthermore, it can assist the manufacturing or purification process if the stability and water solubility of the sarsasapogenin can be controlled.

In principle, the water solubility of polymorphic forms of an organic compound is not necessarily the same for all forms. Therefore, the use of specific crystalline forms or habits can offer useful control of the water solubility. In the case of sparingly watersoluble compounds such as sarsasapogenin, even a slight adjustment to the water-solubility by means of an adjustment to the polymorphic form can offer useful processing or biological advantages.

We have examined commercially available sarsasapogenin and have found that it occurs in a specific crystalline form, which we have characterised as form B.

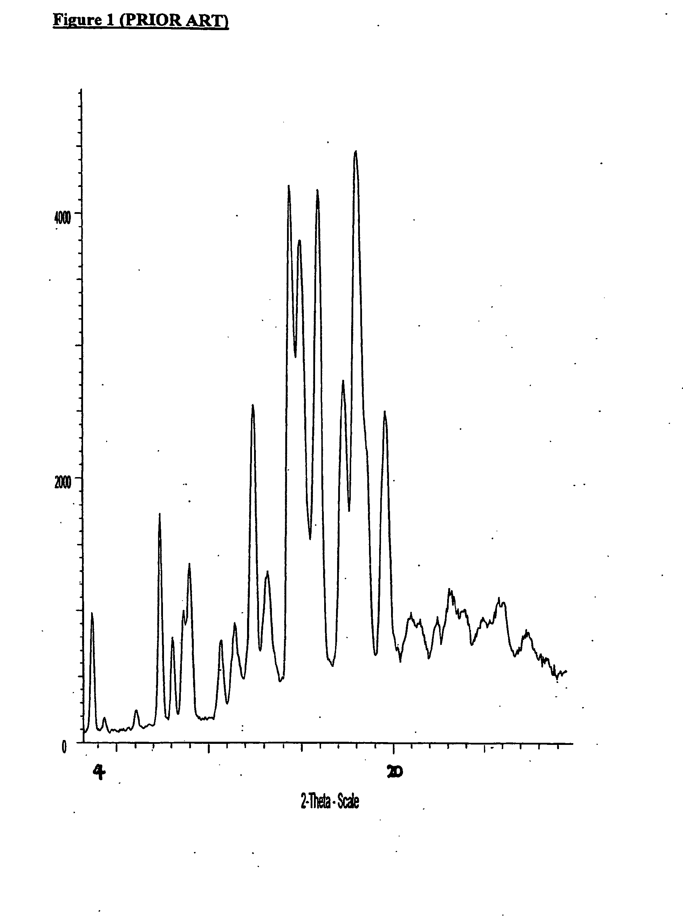

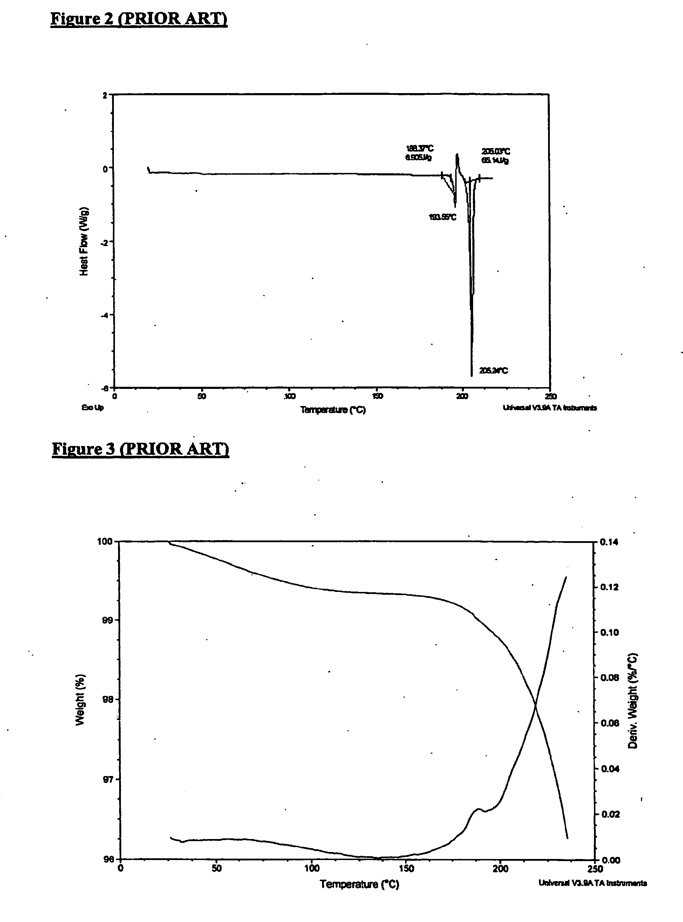

Figure 1 of the accompanying drawings shows an XRPD pattern obtained at λ = 1.5406 Angstroms, Figure 2 shows a differential scanning calorimetry (DSC) trace, and Figure 3 shows a thermogravimetric analysis (TGA) trace, all obtained from a sample of commercially available sarsasapogenin obtained from Steraloids, Inc. (www.steraloids.com). This is an example of form B crystalline sarsasapogenin.

The XRPD pattern was obtained using a Bruker C2 diffractometer equipped with a XYZ stage, a laser video microscope and a HiStar area detector. Typical collection times were 200s. The sealed copper tube (Cu Kα radiation: 1.5406 Angstroms) voltage and current were set at 40 kV and 40 mA respectively. The X-ray optics on the C2 apparatus consisted of a single Gobel mirror coupled with a pinhole collimator of 0.3 mm diameter. The beam divergence (effective size of the X-ray spot) yielded a value of approximately 4 mm.

### Brief Description of the Invention

The present invention is based on our surprising finding that new crystalline forms of sarsasapogenin can be prepared and that a novel amorphous form of sarsasapogenin is expected to be preparable. In particular, but not exclusively, we have found that sarsasapogenin shows a great propensity to crystallise as new solvates or hydrates, with organic solvents or water respectively. Crystallisation with both one or more organic solvent and water has also been observed, and the expression "solvate" used herein includes such forms. Furthermore, the properties of these novel forms of sarsasapogenin offer substantial advantages in the manufacture and use of sarsasapogenin and its derivatives (for example, the 3-esters).

According to a first aspect of the present invention, there is provided sarsasapogenin in crystalline form H (non-hydrate, non-solvate) or in the form of a crystalline solvate or hydrate.

The crystalline sarsasapogenin may be in any one or more of crystalline forms A, C, D, E, F, G, H, I, J, K, L, M, N, O, P, Q, R, Gen1, Gen2, Gen3 and Gen4 as defined herein.

Certain ones of these crystalline forms are solvates, and certain ones are hydrates, as discussed in more detail below.

Thus, according to a second aspect of the present invention, there is provided sarsasapogenin solvate, preferably in crystalline form. The organic solvent used may be selected from those indicated below or other organic solvents or any mixture or combination thereof. The term "organic solvent" is not intended to be restricted to pure solvents or to solvents in which sarsasapogenin is highly soluble at room temperature. It includes, for example, other organic compounds which are liquid at temperatures achievable without degradation of the sarsasapogenin, organic compounds which can be taken up into the sarsasapogenin crystal structure on slurrying the crystals with the compound in liquid form, and mixtures of such compounds. Generally speaking, the criterion is that sarsasapogenin should be able, without undue difficulty in the prevailing laboratory, pilot or commercial environment, to be dissolved or suspended in the organic solvent material and precipitated (and preferably crystallised) therefrom, or to be slurried in crystal form in the organic solvent material.

Furthermore, according to a third aspect of the present invention, there is provided sarsasapogenin hydrate, preferably in crystalline form.

According to a fourth aspect of the present invention, there is provided amorphous sarsasapogenin.

These novel forms of sarsasapogenin and associated methods therefore offer enhanced control of the preparation of pharmaceutical or edible grade sarsasapogenin, and the possibility of preparing pharmaceutical or edible grade sarsasapogenin with improved delivery and bioavailability characteristics.

The crystalline or amorphous material of the present invention may be present substantially free of other forms of sarsasapogenin and/or substantially free of other steroidal sapogenins and/or steroidal saponins.

The crystalline or amorphous material of the present invention may preferably be present in at least about 50% by weight pure form, for example at least about 70% by weight pure form, for example at least about 80% by weight pure form, for example at least about 85% by weight pure form, for example at least about 90% by weight pure form, for example at least about 95% by weight pure form, for example at least about 97% by weight pure form, for example at least about 98% by weight pure form.

The materials according to the present invention may be present in any suitable physical form, for example as an isolated dry solid, which may be flowable or non-flowable, an isolated wet solid, or in a liquid medium such as a crystal slurry.

Any of the materials according to the present invention may if desired be present in admixture with one or more other materials according to the present invention, another form of sarsasapogenin, any other biologically active material, any biologically inactive material, or any combination thereof. The said other form of sarsasapogenin, when present, may be crystalline form B.

The present invention further provides methods of adjusting the crystalline form of sarsasapogenin between the forms A, B, C, D, E, F, G, H, I, J, K, L, M, N, O, P, Q, R, Gen1, Gen2, Gen3 and Gen4, methods of adjusting the form of sarsasapogenin between its amorphous and crystalline forms, and methods of adjusting the hydration or solvation level of sarsasapogenin.

The present invention also provides methods for preparing the materials of the present invention, preferably by precipitation from a solution of sarsasapogenin in an appropriate organic solvent or solvent mixture, optionally in the presence of water. Such a method may include the use of crystallisation modifiers, as will be known to those skilled in this art. This precipitation method can be used to purify sarsasapogenin. In cases where the precipitated material is a solvate or hydrate, this may be converted to unsolvated and unhydrated sarsasapogenin by further steps.

The present invention therefore further provides a method for purification of sarsasapogenin, particularly but not exclusively on a commercial manufacturing scale, which comprises forming hydrated sarsasapogenin crystals in form C and subsequently drying the hydrated sarsasapogenin crystals, preferably at a temperature below about 80°C, more preferably below about 70°C, more preferably below about 60°C, to form relatively pure substantially non-solvated non-hydrated crystalline sarsasapogenin.

The present invention provides an alternative method for purification of sarsasapogenin, particularly but not exclusively on a commercial manufacturing scale, which comprises dissolving sarsasapogenin (including any solvated, hydrated, crystalline or amorphous form thereof) in a mixed alkane/ketone solvent, preferably in the absence or substantial absence of water, and precipitating sarsasapogenin from the resulting solution as relatively pure substantially non-solvated non-hydrated crystalline sarsasapogenin. Generally, the desired end product will precipitate directly from such a solvent.

The expression "relatively pure" in the context of these purification methods refers to a level of purity higher than the starting material.

The present invention further provides a process for obtaining pharmaceutical or edible grade sarsasapogenin or a derivative thereof (for example, a 3-ester derivative such as a 3-carboxylic acid ester derivative), wherein at least one step of the process includes preparing sarsasapogenin in one or more of the forms according to the present invention or wherein the process comprises a purification method according to the present invention. The sarsasapogenin or derivative thereof may be prepared in any suitable level of solvation or hydration and in any suitable physical form, for example as an isolated dry solid or in a liquid medium such as a crystal slurry.

The resultant pharmaceutical or edible grade sarsasapogenin or derivative thereof may be subsequently formulated into a suitable medicament, foodstuff, food supplement, beverage or beverage supplement form. Such formulation may be performed in conventional manner.

The novel forms of sarsasapogenin provided by the present invention possess a number of advantages over the known form, particularly in terms of their stability and handling characteristics. These advantages are applicable to one or more of the manufacturing, purification, formulation and storage phases of the marketed sarsasapogenin compositions (which includes compositions containing derivatives of sarsasapogenin such as its 3-ester derivatives) and/or to the delivery of the sarsasapogenin or derivative thereof from the composition to the human or non-human animal patient for achieving the desired pharmacological effect.

The present invention also provides medicaments, foodstuffs, food supplements, beverages and beverage supplements containing the materials of the present invention, methods of preparing the medicaments, foodstuffs, food supplements, beverages and beverage supplements, uses of the said materials in the preparation of the medicaments, foodstuffs, food supplements, beverages and beverage supplements, and uses of the medicaments, foodstuffs, food supplements, beverages and beverage supplements in human and veterinary medicine and in non-therapeutic human and non-human animal treatments.

### Detailed Description of the Invention

### Solvates

Any organic solvent may in principle be used to prepare a solvate of sarsasapogenin, and we have found that a wide range of solvents produce good results.

As mentioned above, a solvate can include also water together with the one or more organic solvent.

While it is believed that the prior art crystalline form of sarsasapogenin was not an organic solvate, we recognise that the prior art does teach crystallisation of sarsasapogenin from certain organic solvents, particularly acetone. We therefore declare that, if it is shown or considered that an item of prior art fulfils the legal requirement of a prior disclosure of an ethanol solvate of sarsasapogenin or an acetone solvate of sarsasapogenin or any other specific solvate(s) of sarsasapogenin, or renders such a solvate obvious under the relevant legal test, then we reserve the right to insert a disclaimer of such a solvate in the claims of this application and subsequent patent.

The relative molar solvation levels of the solvates according to the present invention can be at, above or below unity.

We have found that solvate formation is efficient if the solvent contains at least one electronegative heteroatom such as oxygen or nitrogen, or a conjugated or aromatic system of unsaturated carbon-carbon bonds.

We have also found that solvates of sarsasapogenin can be obtained which incorporate more than one organic solvent in the crystal structure, or which incorporate one or more organic solvent and water in the crystal structure.

Thus, for example, the solvent may be selected from ketones, alcohols, ethers, esters, aromatic solvents, and, where possible, mixtures thereof with each other, with other organic solvents and/or with water.

### Ketones

Any ketone having a suitable boiling point may be used. There may particularly be mentioned dialkyl ketones, more particularly dialkyl ketones in which the alkyl groups, which may be the same or different, are selected from straight or branched alkyl groups containing from 1 to about 8 carbon atoms (e.g. di-C₁ to C₆ alkyl ketones or C₁ to C₆ alkyl-C₁ to C₆ alkyl-ketones such as acetone, methyl ethyl ketone or methyl iso-butyl ketone).

The ketone may optionally be substituted with one or more substituent. Such substituent may suitably be of a relatively small size in comparison with the ketone molecule, so that solvate formation is not prevented. Examples of suitable substituent groups include heteroatom-containing groups such as N-atom or O-atom containing groups, for example amino or alkoxy (e.g. C₁ to C₆ alkoxy) groups.

We have recrystallised sarsasapogenin from acetone to obtain crystalline sarsasapogenin hemi acetone solvate, which we have termed "form A". Surprisingly, we have found that this sarsasapogenin solvate initially crystallises as the mono-acetone solvate, which then loses half a mole of acetone upon filtration and initial drying.

The hemi acetone solvate shows reasonable stability with respect to further drying, but may be desolvated by heating at about 80-100°C to furnish the known non-solvated form which we have termed form B.

Although such a process has been shown to be useful for the formation of form B, the requirement to heat the product at high temperatures is disadvantageous from the point of view of a large scale manufacuturing process.

For the preparation of sarsasapogenin in its non-solvated form, we have surprisingly found that the desolvation temperatures of the solvates with higher ketones, such as methyl ethyl ketone solvate or methyl *iso*-butyl ketone, are lower than for acetone.

### Alcohols

Any alcohol having a suitable boiling point may be used. There may particularly be mentioned alkyl alcohols, more particularly alkyl mono-ols in which the alkyl group is selected from straight or branched alkyl groups containing from 1 to about 8 carbon atoms, for example containing 2 to about 8 carbon atoms, for example containing from 3 to about 8 caron atoms, for example containing from 4 to about 8 carbon atoms (e.g. C₁ to C₆ alkyl mono-ols such as methanol, ethanol, n-propanol, n-butanol, iso-propanol and *tert*-butanol). The alcohol may optionally be substituted with one or more substituent. Such substituent may suitably be of a relatively small size in comparison with the alcohol molecule, so that solvate formation is not prevented. Examples of suitable substituent groups include heteroatom-containing groups such as N-atom or O-atom containing groups, for example amino, alkoxy (e.g. C₁ to C₆ alkoxy) or alkoxy-substituted alkoxy (e.g. C₁ to C₆ alkoxy-substituted C₁ to C₆ alkoxy) groups.

For example, we have found, surprisingly, that sarsasapogenin forms solvates with methanol, ethanol, *n*-propanol, *iso*-propanol, *n*-butanol, *tert*-butanol, 3-methyl-1-butanol, aminoethanol or diethyleneglycol monomethyl ether.

In contrast to the acetone solvate mentioned above, we have found that the solvates of sarsasapogenin that have the Gen1 crystalline form as defined below, for example the solvates with *n*-propanol or *n*-butanol, maintain their initial solvate stoichiometry on filtration and drying. However, the ethanol solvate appears to show variable stoichiometry, in that an initial bis-ethanol solvate can desolvate to the hemisolvate on drying.

We have further found that mixed solvates of sarsasapogenin can be prepared in which at least one of the solvating components is an alcohol. Examples of such forms of sarsasapogenin include mixed alkyl mono-ol and water solvates and mixed alkyl mono-ol and ether solvates, such as sarsasapogenin mono-methanol solvate monohydrate and sarsasapogenin mono-methanol hemi-tetrahydrofuran solvate.

### Ethers

Any ether having a suitable boiling point may be used. There may particularly be mentioned dialkyl ethers, more particularly dialkyl ethers in which the alkyl groups, which may be the same or different, are selected from straight or branched alkyl groups containing from 1 to about 8 carbon atoms (e.g. di-C₁ to C₆ alkyl-ethers or C₁ to C₆ alkyl-C₁ to C₆ alkyl-ethers such as *tert*-butyl methyl ether). Cyclic ethers may also be mentioned, such as cyclic alkylene oxides in which the alkylene group is selected from straight or branched alkylene groups containing from 2 to about 8 carbon atoms (e.g. cyclic C₂ to C₆ alkylene oxides such as tetrahydrofuran (THF)).

The ether may optionally be substituted with one or more substituent. Such substituent may suitably be of a relatively small size in comparison with the ether molecule, so that solvate formation is not prevented. Examples of suitable substituent groups include heteroatom-containing groups such as N-atom or O-atom containing groups, for example amino or alkoxy (e.g. C₁ to C₆ alkoxy) groups.

### Esters

Any ester having a suitable boiling point may be used. There may particularly be mentioned alkyl alkanoate esters, particularly alkyl alkanoate esters in which the alkyl groups, which may be the same or different, are selected from straight or branched alkyl groups containing from 1 to about 8 carbon atoms (e.g. C₁ to C₆ alkyl formates such as ethyl formate or C₁ to C₆ alkyl acetates such as *n*-butyl acetate, *iso*-propyl actetate or ethyl acetate).

The ester may optionally be substituted with one or more substituent. Such substituent may suitably be of a relatively small size in comparison with the ester molecule, so that solvate formation is not prevented. Examples of suitable substituent groups include heteroatom-containing groups such as N-atom or O-atom containing groups, for example amino or alkoxy (e.g. C₁ to C₆ alkoxy) groups.

### Hydrocarbon Solvents

Any hydrocarbon solvent having a suitable boiling point may be used. There may particularly be mentioned aromatic solvents containing one or more phenyl group, more particularly mono- or poly-alkyl benzenes in which the alkyl group(s), which when more than one is present may be the same or different, is/are selected from straight or branched alkyl groups containing from 1 to about 8 carbon atoms (e.g. mono- or poly-C₁ to C₆ alkyl benzenes such as *o*-xylene, *m*-xylene, *p*-xylene, toluene or cumene).

The hydrocarbon solvent may optionally be substituted with one or more substituent. Such substituent may suitably be of a relatively small size in comparison with the hydrocarbon molecule, so that solvate formation is not prevented. Examples of suitable substituent groups include heteroatom-containing groups such as N-atom or O-atom containing groups, for example amino or alkoxy (e.g. C₁ to C₆ alkoxy) groups. A suitable example of a hetero-substituted hydrocarbon solvent is phenethylamine (2-phenylethylamine).

### Hydrates

We have found that sarsasapogenin hydrates can be formed, sometimes remarkably easily.

The relative molar hydration levels of the hydrates can be at, above or below unity.

For example, we have surprisingly found that, when sarsasapogenin is recrystallised from aqueous organic solvents such as water mixtures with the water-miscible solvents mentioned above, a crystalline hydrate that contains about half a mole of water per mole of sarsasapogenin, for example between about 0.25 and 0.75 mole of water per mole of sarsasapogenin, typically from about 0.3 to about 0.5 of a mole of water, can be isolated (nominally "sarsasapogenin hemihydrate"), in forms which we have termed "form C" and "form I". This was confirmed by water determination by Karl Fischer analysis and the elemental analaytical data obtained.

For further details of form C and form I crystalline sarsasapogenin hydrate, see the discussion below.

### Crystalline Form A

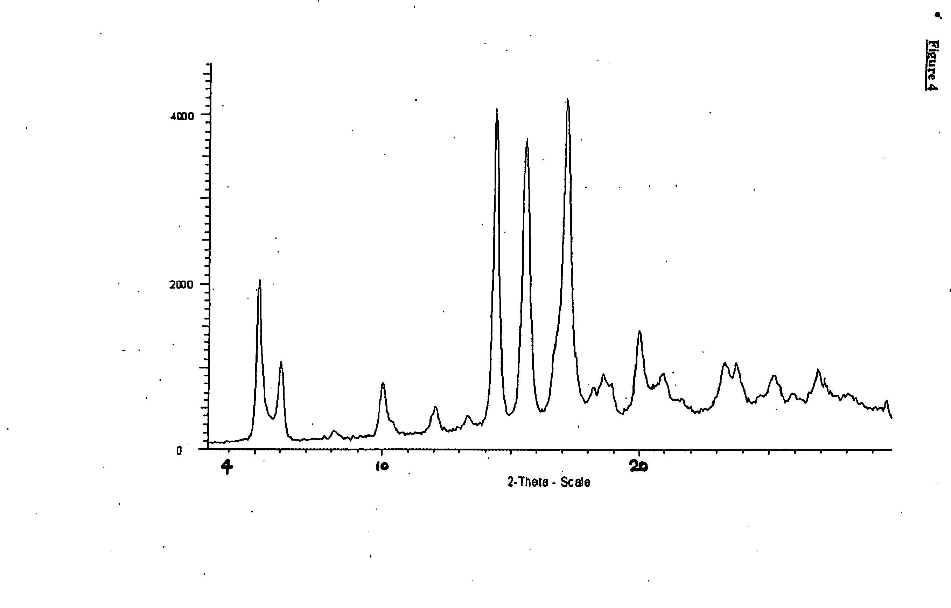

The term "crystalline form A" used herein means that crystalline form of sarsasapogenin which has an XRPD pattern substantially as shown in Figure 4 of the accompanying drawings (λ = 1.5406 Angstroms), or which has unit cell dimensions, angles and space group substantially as set forth for form A in Table 1 below.

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 4, using the Bragg equation.

Crystalline form A is an example of the more general crystalline form of sarsasapogenin characterised by us as form Gen1 on the basis of the unit cell dimensions, angles and space group observed through single crystal X-ray crystallography, as described in more detail below.

The form A material on which Figure 4 was obtained is crystalline acetone hemisolvate, and the form A material on which the data in Table 1 were obtained is crystalline acetone monosolvate. Both materials can be prepared by recrystallisation of commercially available sarsasapogenin from acetone. The hemisolvate is obtained by desolvation on drying of the initial monosolvate form. Further details are given in Example 1 below.

### Crystalline Form B

The term "crystalline form B" used herein means that crystalline form of sarsasapogenin which has an XRPD pattern substantially as shown in Figure 1 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 1, using the Bragg equation.

The form B material was commercially available sarsasapogenin obtained from Steraloids Inc. DSC (Figure 2), TGA (Figure 3), residual solvent and Karl Fischer analysis confirmed that the material was neither hydrated nor solvated. Further details are given in Examples 2 and 3 below.

### Crystalline Form C

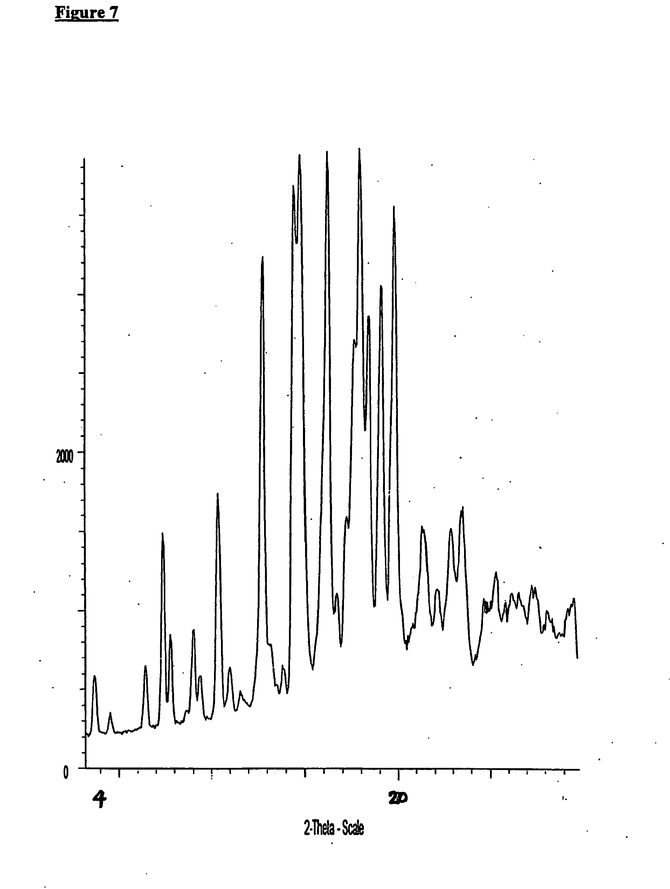

The term "crystalline form C" used herein means that crystalline form of sarsasapogenin which has an XRPD pattern substantially as shown in Figure 7 of the accompanying drawings (λ =1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 7, using the Bragg equation.

On the basis of our observations that the hydrates of sarsasapogenin exist in a wide range of stoichiometries, generally nominally hemihydrates but varying substantially either side of that stoichiometry, we conclude that crystalline form C is an example of the more general crystalline form of sarsasapogenin characterised by us as form Gen2 on the basis of the unit cell dimensions, angles and space group observed through single crystal X-ray crystallography, as described in more detail below.

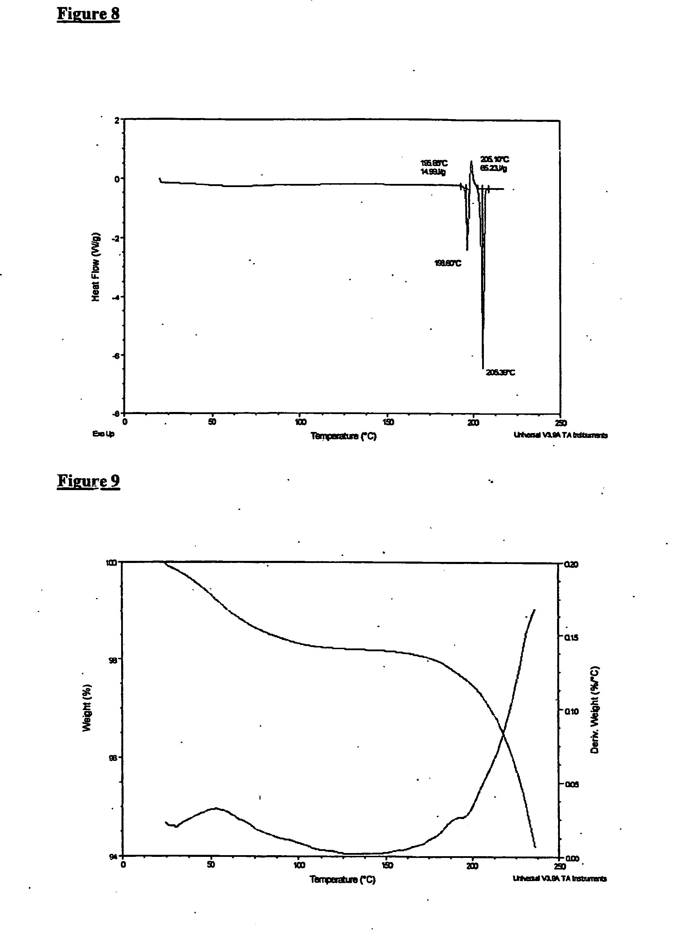

The form C material may be prepared by solvent mediated transformation of commercially available sarsasapogenin from aqueous solvent mixtures in which the solvent is selected from any of those available for formation of sarsasapogenin solvates according to this invention. The aqueous solvent mixtures thus include, by way of example but without limitation: 9:1 v/v acetone/water, 9:1 v/v n-butyl acetate/water, 9:1 v/v *tert*-butyl methyl ether/water, 9:1 v/v cumene/water, 9:1 v/v ethyl acetate/water, 9:1 v/v ethyl formate/water, 9:1 v/v methyl ethyl ketone/water, 9:1 v/v methyl *iso*-butyl ketone/water, 9:1 v/v *iso*-propanol/water, 9:1 v/v *iso*-propyl acetate/water, 9:1 v/v tetrahydrofuran/water, 3:1 v/v tetrahydrofuran/water, 1:1 v/v water/tetrahydrofuran, 3:1 v/v water/tetrahydrofuran, 3:1 v/v water/ethanol, or 3:1 v/v water/methanol. Differential scanning calorimetry (Figure 8), thermogravimetric analysis (Figure 9) and Karl Fischer analysis confirmed that the crystalline form C that we have obtained is hydrated. Further details are given in Examples 4 to 6 below.

### Crystalline Form D

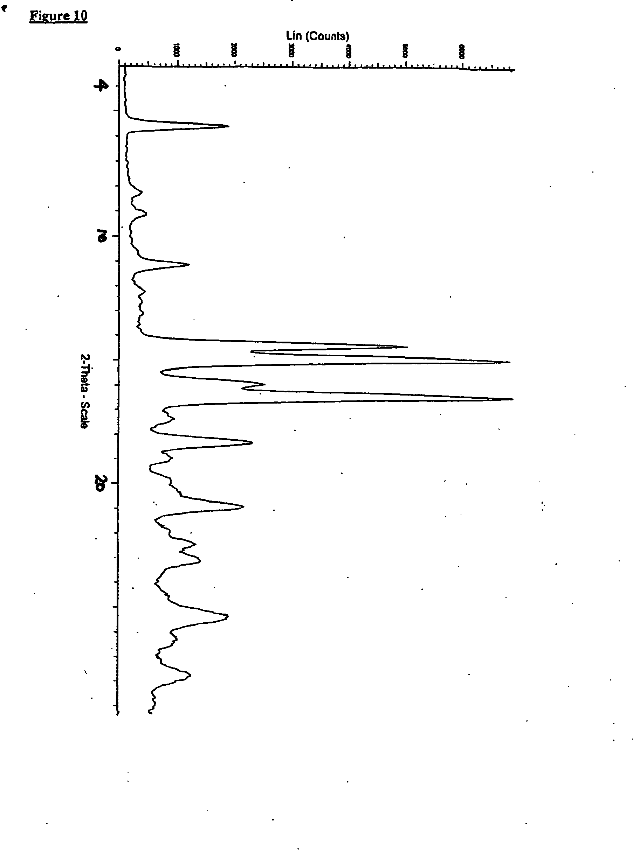

The term "crystalline form D" used herein means that crystalline form of sarsasapogenin which has an XRPD pattern substantially as shown in Figure 10 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 10, using the Bragg equation.

Crystalline form D is an example of the more general crystalline form of sarsasapogenin characterised by us as form Gen3 on the basis of the unit cell dimensions, angles and space group observed through single crystal X-ray crystallography, as described in more detail below.

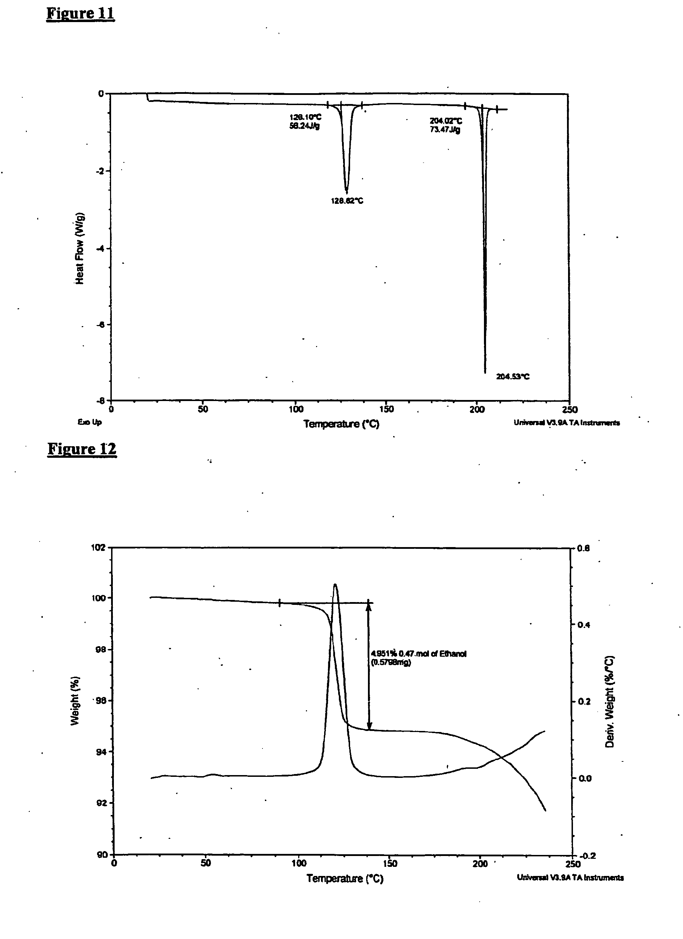

Differential scanning calorimetry, (Figure 11) thermogravimetric analysis (Figure 12) and NMR analysis confirmed that the crystalline form D that we have obtained is a hemi-ethanol solvate of sarsasapogenin. The form D material may be prepared by a recrystallisation of sarsasapogenin from ethanol, ethanol-water mixtures or ethanol-solvent mixtures. Further details are given in Examples 7 to 9 below.

The form D material on which Figures 10 to 12 were obtained is crystalline ethanol hemisolvate, and the form D material on which the data in Table 1 were obtained is crystalline ethanol bis-solvate. Both materials can be prepared by recrystallisation of commercially available sarsasapogenin from ethanol, ethanol-water mixtures or ethanol-solvent mixtures. The hemisolvate is obtained by desolvation on drying of the initial bis-solvate form.

### Crystalline Form E

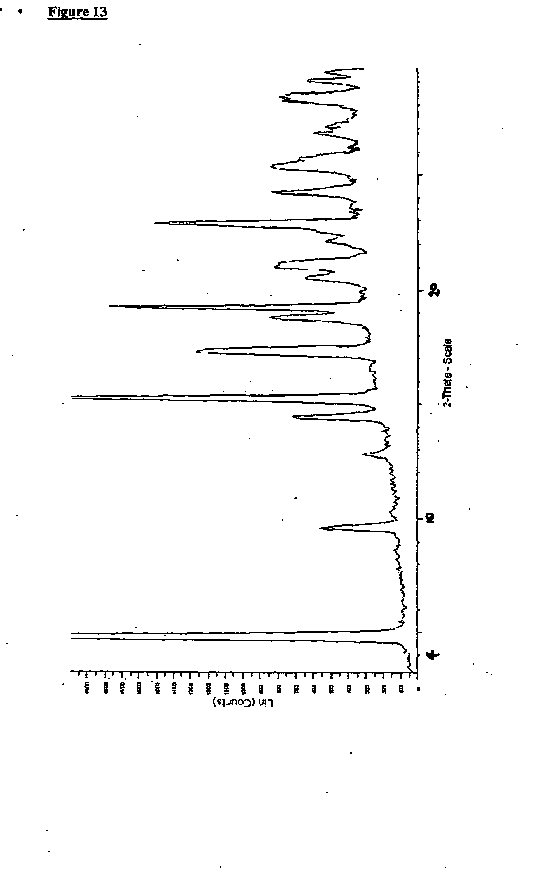

The term "crystalline form E" used herein means that crystalline form of sarsasapogenin which has an XRPD pattern substantially as shown in Figure 13 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 13, using the Bragg equation.

Crystalline form E is another example of the Gen1 form of crystalline sarsasapogenin.

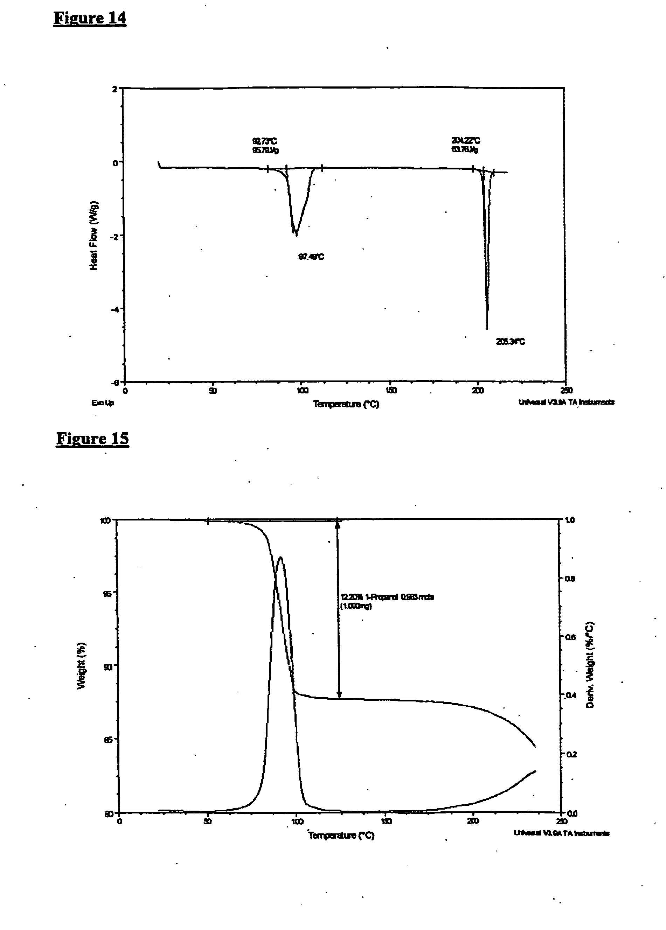

Differential scanning calorimetry (Figure 14), thermogravimetric analysis (Figure 15) and NMR analysis confirmed that the crystalline form E that we have obtained is an n-propanol solvate of sarsasapogenin. The form E material may be prepared in relatively pure form by recrystallisation of sarsasapogenin from n-propanol. Further details are given in Example 12 below.

### Crystalline form F

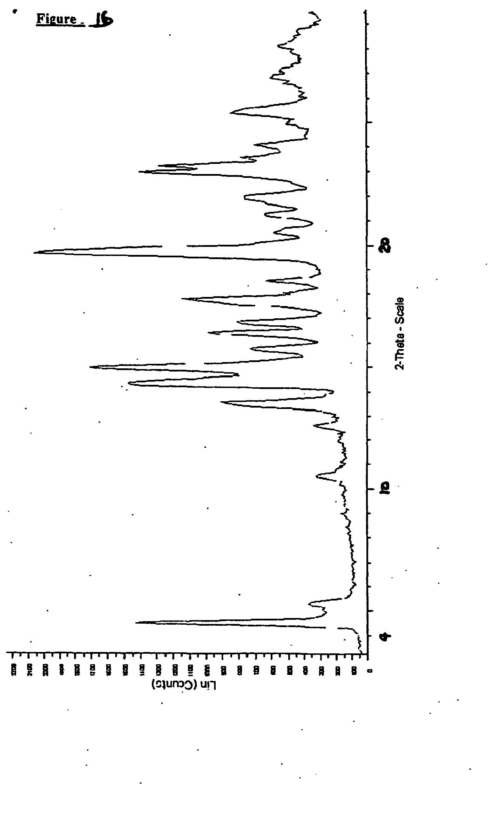

The term "crystalline form F" used herein means that crystalline form of sarsasapogenin which has an XRPD pattern substantially as shown in Figure 16 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 16, using the Bragg equation.

Crystalline form F is another example of the Gen3 form of crystalline sarsasapogenin.

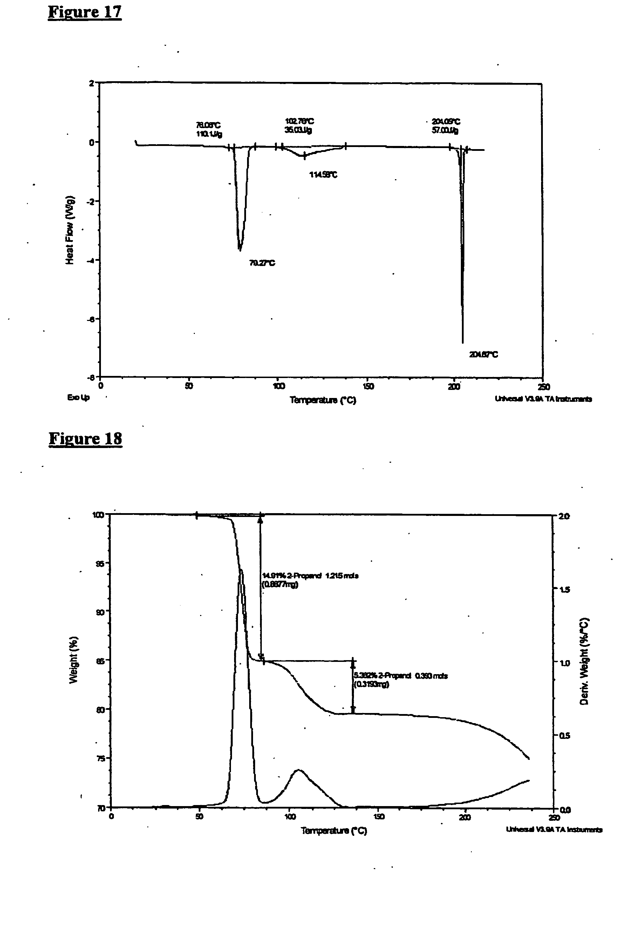

Differential scanning calorimetry (Figure 17), thermogravimetric analysis (Figure 18) and NMR analysis confirmed that the crystalline form F that we have obtained is an iso-propanol solvate of sarsasapogenin. The form F material may be prepared in relatively pure form by recrystallisation of sarsasapogenin from *iso*-propanol. Further details are given in Example 13 below.

### Crystalline form G

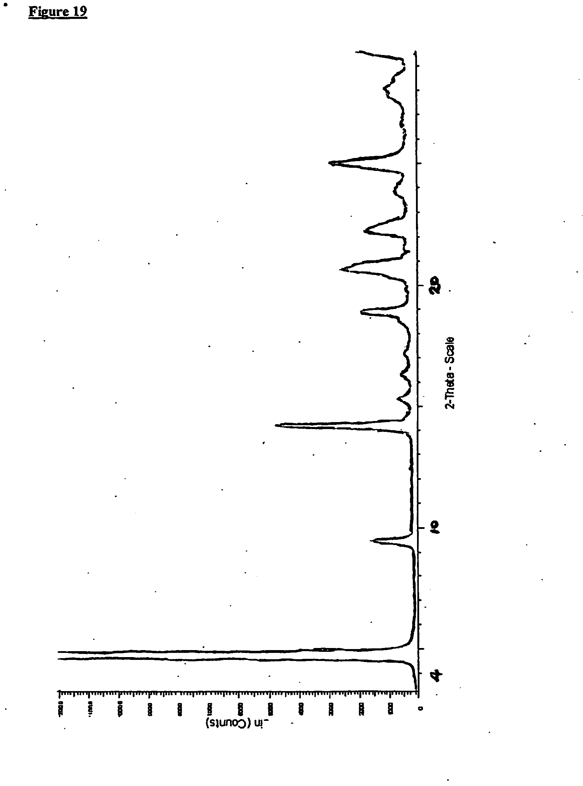

The term "crystalline form G" used herein means that crystalline form of sarsasapogenin which has an XRPD pattern substantially as shown in Figure 19 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 19, using the Bragg equation.

Crystalline form G is another example of the Gen1 form of crystalline sarsasapogenin.

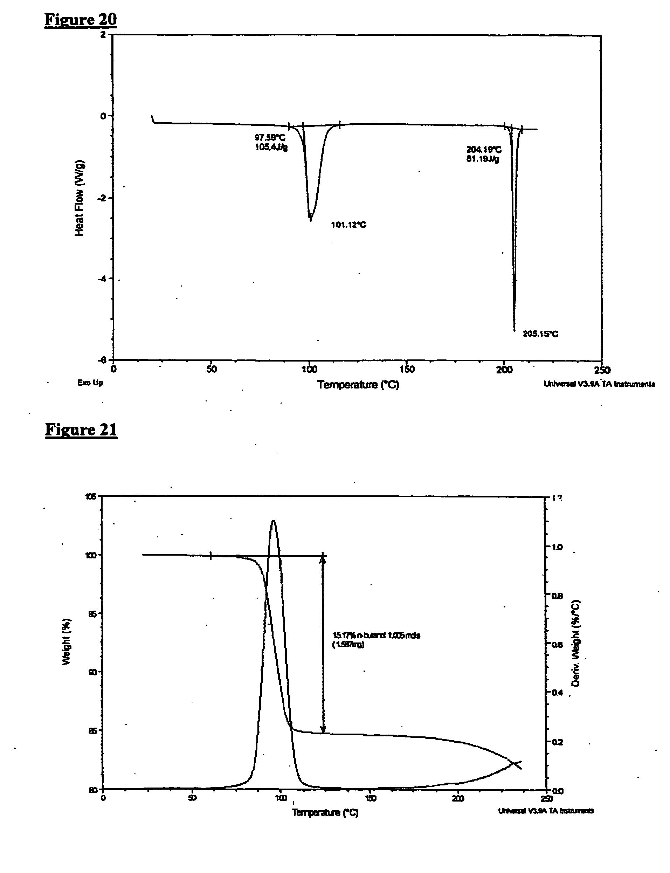

Differential scanning calorimetry (Figure 20), thermogravimetric analysis (Figure 21) and NMR analysis confirmed that the crystalline form G that we have obtained is an *n-*butanol solvate of sarsasapogenin. The form G material may be prepared in relatively pure form by recrystallisation of sarsasapogenin from n-butanol. Further details are given in Example 11 below.

### Crystalline form H

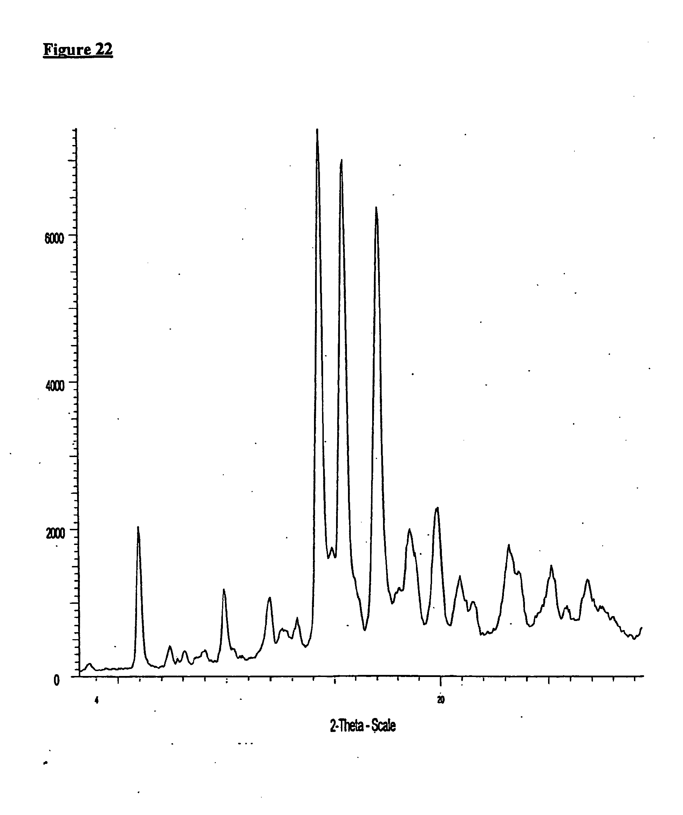

The term "crystalline form H" used herein means that crystalline form of sarsasapogenin which has an XRPD pattern substantially as shown in Figure 22 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 22, using the Bragg equation.

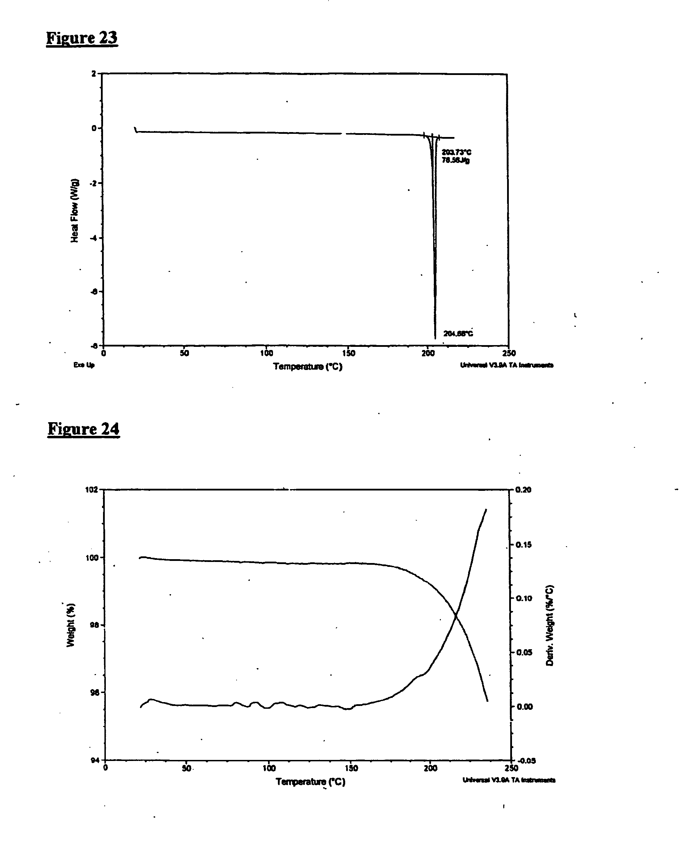

Differential scanning calorimetry (Figure 23) and thermogravimetric analysis (Figure 24) confirmed that this form is not hydrated or solvated.

Form H may be prepared by heating the ethanol solvate above 130°C. Further details are given in Example 10 below.

### Crystalline form I

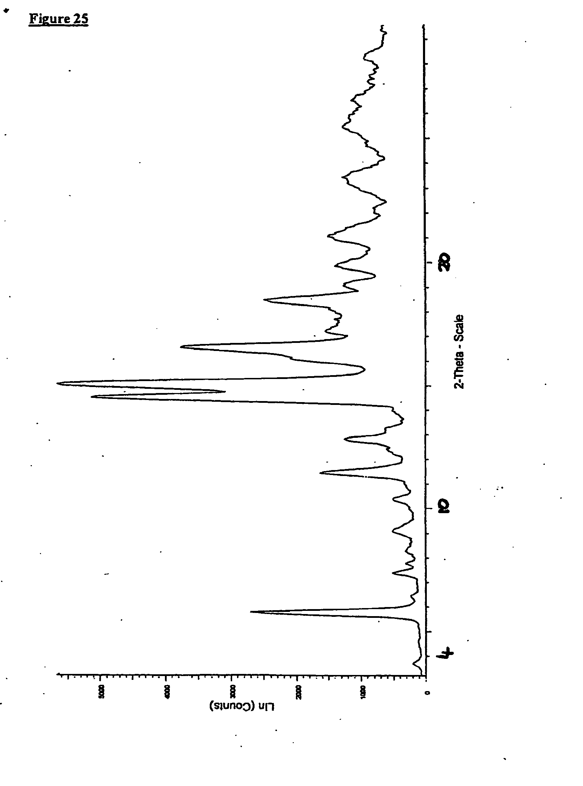

The term "crystalline form I" used herein means that crystalline form of sarsasapogenin which has an XRPD pattern substantially as shown in Figure 25 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 25, using the Bragg equation.

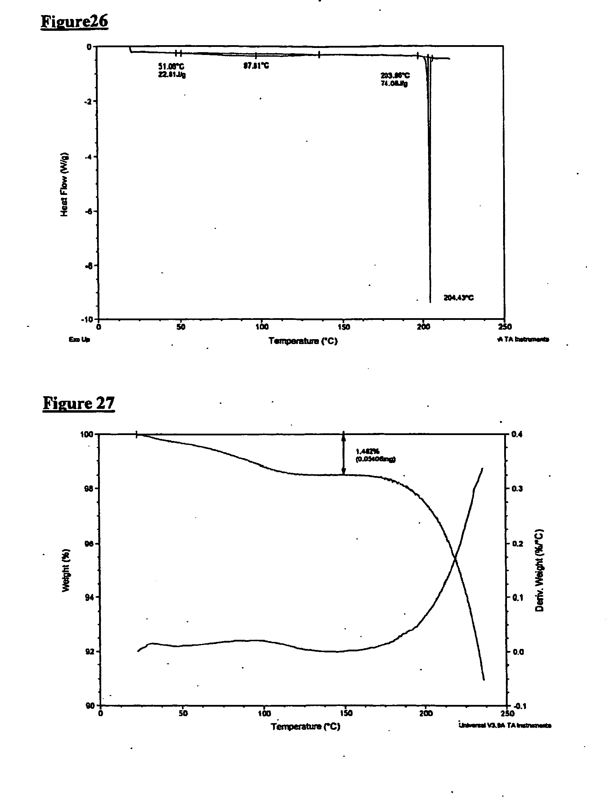

Differential scanning calorimetry (Figure 26), thermogravimetric analysis (Figure 27) and Karl Fischer analysis confirmed that the crystalline form I is a hydrate of sarsasapogenin. Further details are given in Example 14 below.

### Crystalline form J

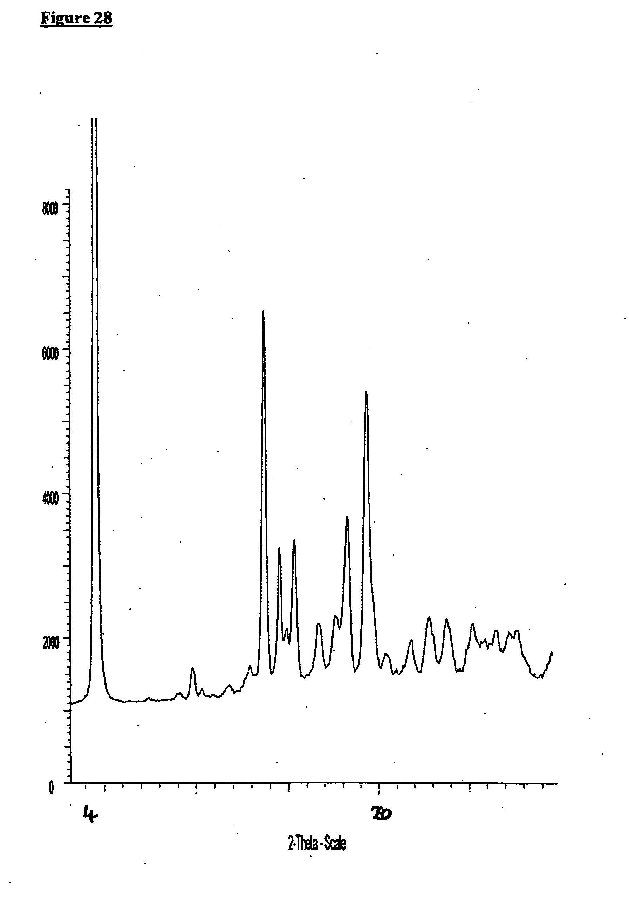

The term "crystalline form J" used herein means that crystalline form of sarsasapogenin which has an XRPD pattern substantially as shown in Figure 28 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 28, using the Bragg equation.

Crystalline form J is another example of the Gen 1 form of crystalline sarsasapogenin.

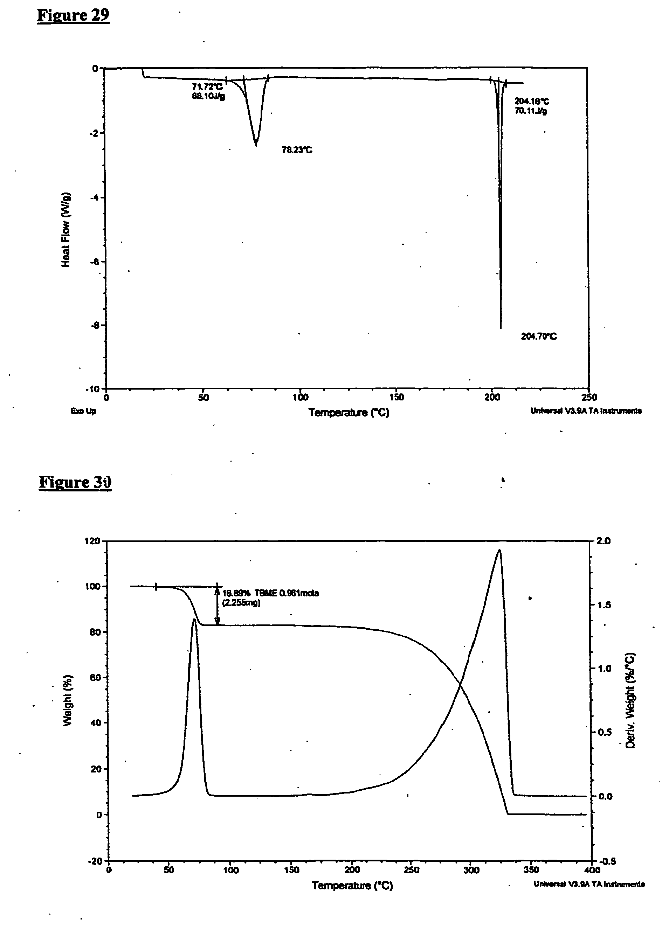

Differential scanning calorimetry (Figure 29), thermogravimetric analysis (Figure 30) and NMR analysis confirmed that the crystalline form J is the *tert*-butyl methyl ether solvate of sarsasapogenin. The form J material may be prepared in relatively pure form by recrystallisation of sarsasapogenin from *tert*-butyl methyl ether. Further details are given in Example 15 below.

### Crystalline form K

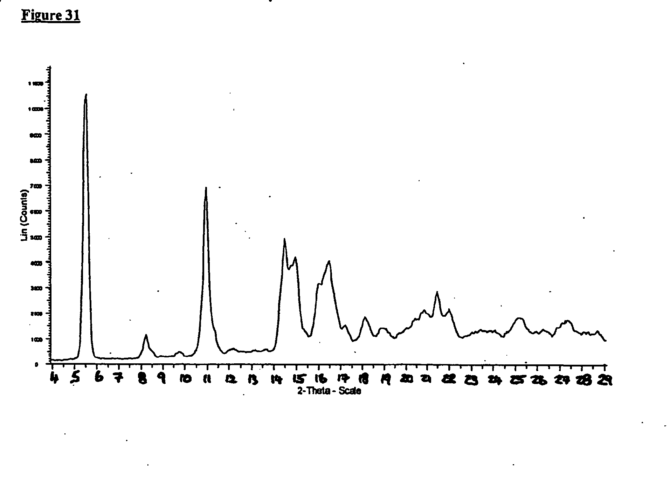

The term "crystalline form K" used herein means that crystalline form of sarsasapogenin which has an XRPD pattern substantially as shown in Figure 31 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 31, using the Bragg equation.

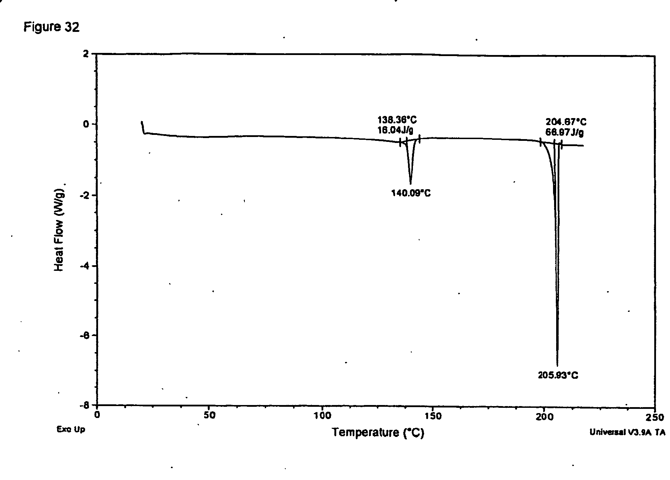

Differential scanning calorimetry (Figure 32) and NMR analysis confirmed that the crystalline form K is the hemi-methanol solvate of sarsasapogenin. The form K material may be prepared in relatively pure form by recrystallisation of sarsasapogenin from methanol. Further details are given in Example 16 below.

### Crystalline form L

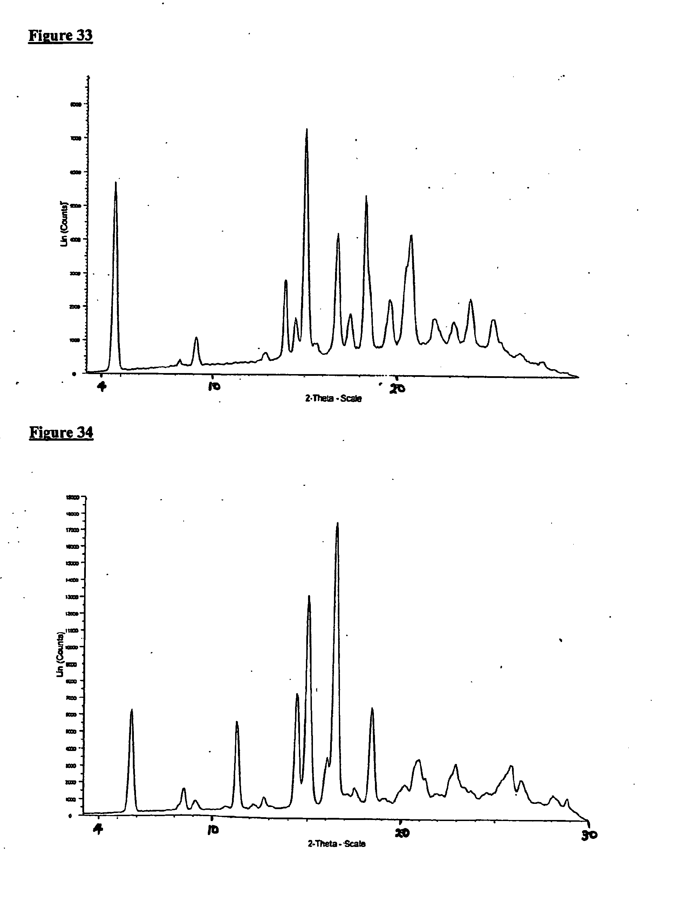

The term "crystalline form L" used herein means that crystalline form of sarsasapogenin which has an XRPD pattern substantially as shown in Figure 33 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 33, using the Bragg equation.

Crystalline form L is another example of the Gen1 form of crystalline sarsasapogenin.

This XRPD data, as well as single crystal X-ray crystallography studies, show that the crystalline form L is a 3-methyl-1-butanol solvate of sarsasapogenin. The form L material may be prepared in relatively pure form by recrystallisation of sarsasapogenin from 3-methyl-1-butanol.

### Crystalline form M

The term "crystalline form M" used herein means that crystalline form of sarsasapogenin which has an XRPD pattern substantially as shown in Figure 34 of the accompanying drawings (λ = 1.5406 Angstroms), or which has unit cell dimensions, angles and space group substantially as set forth for form M in Table 1 below.

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 34, using the Bragg equation.

This XRPD data, as well as single crystal X-ray crystallography studies, show that the crystalline form M is a mixed mono-methanol hemi-THF solvate of sarsasapogenin. The form M material may be prepared in relatively pure form by recrystallisation of sarsasapogenin from a mixture of methanol and tetrahydrofuran (THF) containing one mole equivalent of raffinose.

### Crystalline form N

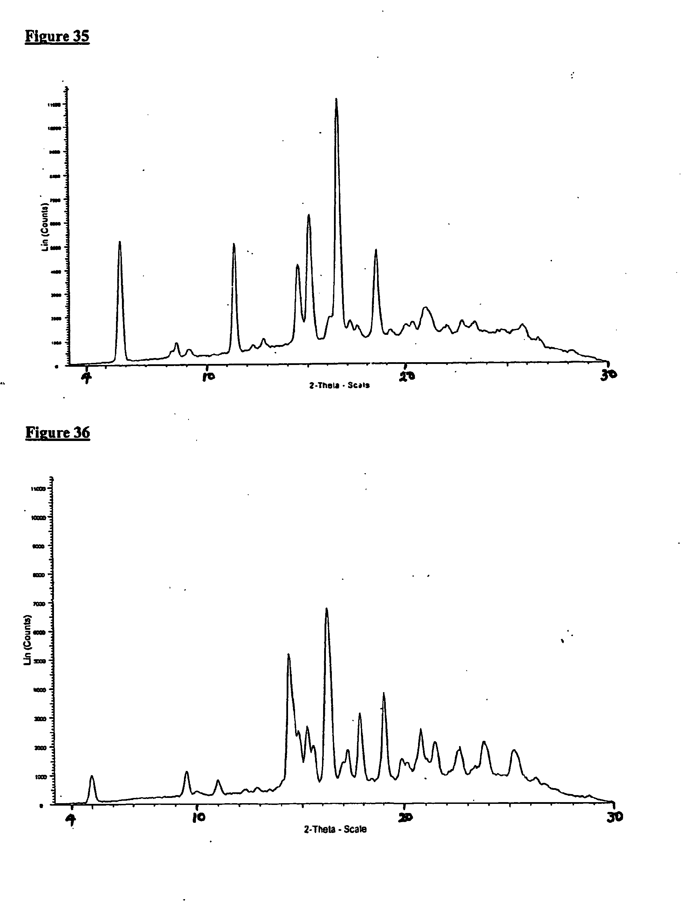

The term "crystalline form N" used herein means that crystalline form of sarsasapogenin which has an XRPD pattern substantially as shown in Figure 35 of the accompanying drawings (λ = 1.5406 Angstroms), or which has unit cell dimensions, angles and space group substantially as set forth for form N in Table 1 below.

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 35, using the Bragg equation.

This XRPD data, as well as single crystal X-ray crystallography studies, show that the crystalline form N is a mixed mono-methanol monohydrate solvate of sarsasapogenin. The form N material may be prepared in relatively pure form by recrystallisation of sarsasapogenin from a mixture of methanol and tetrahydrofuran (THF) containing one mole equivalent of sucrose.

### Crystalline form O

The term "crystalline form O" used herein means that crystalline form of sarsasapogenin which has an XRPD pattern substantially as shown in Figure 36 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 36, using the Bragg equation.

Crystalline form O is another example of the Gen3 form of crystalline sarsasapogenin.

This XRPD data, as well as single crystal X-ray crystallography studies, show that the crystalline form O is a diethyleneglycol monomethyl ether solvate of sarsasapogenin. The form O material may be prepared in relatively pure form by recrystallisation of sarsasapogenin from diethyleneglycol monomethyl ether.

### Crystalline form P

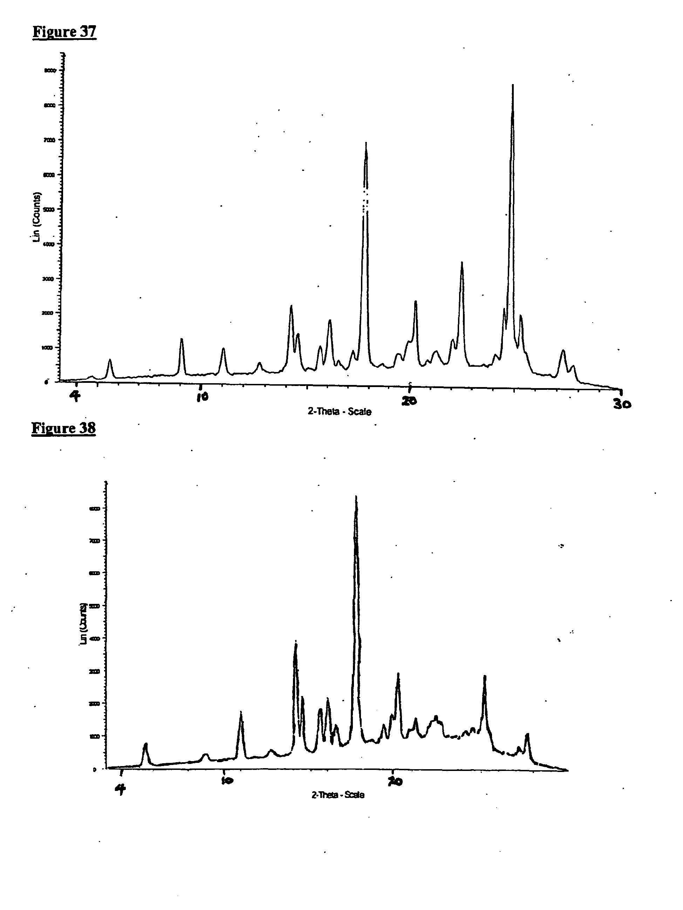

The term "crystalline form P" used herein means that crystalline form of sarsasapogenin which has an XRPD pattern substantially as shown in Figure 37 of the accompanying drawings (λ = 1.5406 Angstroms), or which has unit cell dimensions, angles and space group substantially as set forth for form P in Table 1 below.

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 37, using the Bragg equation.

This XRPD data, as well as single crystal X-ray crystallography studies, show that the crystalline form P is an aminoethanol solvate of sarsasapogenin. The form P material may be prepared in relatively pure form by recrystallisation of sarsasapogenin from aminoethanol.

### Crystalline form Q

The term "crystalline form Q" used herein means that crystalline form of sarsasapogenin which has an XRPD pattern substantially as shown in Figure 38 of the accompanying drawings (λ = 1.5406 Angstroms), or which has unit cell dimensions, angles and space group substantially as set forth for form Q in Table 1 below..

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees or that portion of the range covered by the XRPD equipment, for example 5 to 30 degrees, may generally be considered characteristic or diagnostic of the crystalline form, subject however to standard practice in crystallography.

The d-spacings may readily be calculated from the information in Figure 38, using the Bragg equation.

This XRPD data, as well as single crystal X-ray crystallography studies, show that the crystalline form Q is a phenylethylamine solvate of sarsasapogenin. The form Q material may be prepared in relatively pure form by recrystallisation of sarsasapogenin from phenylethylamine.

### Crystalline form R

The term "crystalline form Q" used herein means that crystalline form of sarsasapogenin which has unit cell dimensions, angles and space group substantially as set forth for form R in Table 1 below.

The crystalline form R is a *tert.*-butanol solvate of sarsasapogenin. The form R material may be prepared in relatively pure form by recrystallisation of sarsasapogenin from *tert*.-butanol.

### Amorphous form

It is strongly expected from the above findings and common general knowledge that an amorphous form of sarsasapogenin is also readily achievable. In principle, a molecule of the size of sarsasapogenin needs a relatively long time interval to arrange itself in a crystal lattice formation. If that time is not available to it at the time of crystallisation, or recrystallisation, an amorphous form will inevitably result. Such an amorphous form may extend across the total bulk of the solid, e.g. the precipitated solid, or may be interspersed in the bulk of one or more crystalline forms in the solid, e.g. the precipitated solid.

We have found, in separate work (PCT/GB2005/001635; Example 13) with the 25-epimer of sarsasapogenin (smilagenin), that the amorphous form of smilagenin is obtained by heating 10 g of smilagenin to its melt using a temperature controlled heating mantle until a complete molten liquid was formed. The molten mass was poured into a Dewar containing approximately 150 ml of liquid nitrogen. The sample was decanted into a glass beaker and the liquid nitrogen allowed to evaporate. The sample was then transferred to a glass vial, flushed with dry nitrogen and then sealed. On the basis of the crystallisation properties of sarsasapogenin reported herein, and our knowledge (PCT/GB2005/001635; Examples 1 to 12) that smilagenin also has a family of hydrated and crystalline forms, we strongly expect that a corresponding melting and sudden temperature plunge procedure would yield amorphous sarsasapogenin.

### Gen1, Gen2, Gen3 and Gen4 forms

Single crystal X-ray crystallography studies, obtained using a Bruker-Nonius Kappa CCD diffractometer equipped with an Oxford Cryosystems Cryostream cooling device, have allowed the unit cell dimensions, angles and space group of each crystal form to be studied. In this work, structures were solved with either SIR-97 or SHELXS-97 and refined with SHELXL-97.

This work, reported in Table 1 below, has enabled us to group together different ones of crystal forms A to R as set out above, as well as a THF solvate and a *tert*.-butanol solvate, according to substantial similarity of the unit cell dimensions a, b, c, angles α, β and γ and the space groups.

**Table 1**

| Group | Form | Solvent | a | b | C | α | β | γ | Space Group |
|---|---|---|---|---|---|---|---|---|---|
| | A | Acetone | 10.5943 | 7.4783 | 17.669 | 90 | 99.658 | 90 | P21 |
| | | | (10) | (8) | (2) | | (4) | | |
| | J | TBME | 10.4766 | 7.4136 | 19.2846 | 90 | 95.001 | 90 | P21 |
| | | | (3) | (2) | (7) | | (1) | | |
| | G | n-butanol | 10.5361 | 7.3919 | 18.6599 | 90 | 90.591 | 90 | P21 |
| | | | (4) | (3) | (8) | | (2) | | |
| Gen1 | E | n-propanol | 10.5547 | 7.4233 | 18.2031 | 90 | 90.628 | 90 | P21 |
| | | | (4) | (2) | (7) | | (1) | | |
| | L | 3-methyl | 10.5534 | 7.3618 | 19.0573 | 90 | 95.593 | 90 | P21 |
| | | butanol | (12) | (8) | (21) | | (4) | | |
| Gen2 | C | Water | 49.1440 | 7.6428 | 19.8610 | 90 | 102.389 | 90 | C2 |
| | | | | | | | (8) | | |
| | D | Ethanol | 7.4419 | 10.4628 | 38.5542 | 90 | 90 | 90 | P212-121 |
| | | | (1) | (2) | (7) | | | | |
| Gen3 | O | DGME | 7.3498 | 10.7281 | 35.3233 | 90 | 90 | 90 | P212-121 |
| | | | (12) | (18) | (55) | | | | |
| | F | 2-propanol | 7.4692 | 10.7388 | 38.9445 | 90 | 90 | 90 | P212-121 |
| | | | (1) | (1) | (5) | | | | |
| Gen4 | | t-butanol | 39.4677 | 7.4389 | 10.6503 | 90 | 94.221 | 90 | C2 |
| | R | | (11) | (2) | (3) | | (1) | | |
| M | M | methanol, THF | 7.4607 | 10.5188 | 18.3056 | 81.238 | 82.252 | 90.0- | P1 |
| | | | (1) | (2) | (4) | (1) | (1) | 06 | |
| N | N | methanol, H₂O | 10.7785 | 7.4415 | 31.386 | 90 | 98.56(3) | 90 | P21 |
| | | | (6) | (4) | (2) | | | | |
| P | P | amino-ethanol | 11.0089 | 7.5701 | 16.1972 | 90 | 98.53 | 90 | P21 |
| | | | (3) | (2) | (5) | | | | |
| Q | Q | phenyl-ethylamine | 7.33640 | 34.7431 | 10.7799 | 90 | 90.637 | 90 | P21 |
| | | | (10) | (4) | (2) | | | | |

Dimensions, angles and space group nomenclature and units are according to normal crystallographic convention and usage. Error margins are indicated in brackets in the conventional way.

From Table 1 it is seen that the crystalline forms of sarsasaspogenin according to the present invention fall into at least 8 distinct crystal form groups, identified as Gen1, Gen2, Gen3, Gen4, M, N, P and Q. Gen1 and Gen3 are groups for which more than one distinct example has been identified; Gen2 is the group representing the hydrates, where a range of hydrate forms, nominally hemihydrates but having quite variable stoichiometry between individual samples, have been identified; Gen4 is a group represented by the *tert*.-butanol solvate R but believed likely to contain other examples; M, N, P and Q are groups where to date only one example has been identified, namely in each case the crystalline form bearing the same respective alphabetical letter. It will be noted that, as discussed above, some crystalline forms show variable stoichiometry.

As shown in Table 1, crystalline forms A, E, G, J and L are related and can be considered as representatives of a first general crystalline form of sarsasapogenin solvates, which we will call Gen1 herein.

Crystalline forms D, F and O are related and can be considered as representatives of a second general crystalline form of sarsasapogenin solvates, which we will call Gen3 herein.

Each respective crystal form represented by the hydrates C and the tert.-butanol solvate R is believed to be of broader applicability, and has therefore been designated Gen2 and Gen4.

The crystal forms M, N, P and Q do not appear to be related to themselves or to any other crystalline form of sarsasapogenin or its solvates and hydrates currently known.

### Purification Process

We have found that form C sarsasapogenin may be readily converted into the known non-hydrated non-solvated crystalline form B by drying at temperatures in the range of about 40-60°C or higher, which is substantially lower than that required to convert form A (the acetone hemi solvate) into form B. This observation has enabled us to develop a novel, practical, process for the large scale purification of sarsasapogenin.

As stated above, the present invention thus provides a method for purification of sarsasapogenin, particularly but not exclusively on a commercial manufacturing scale, which comprises forming hydrated sarsasapogenin crystals in form C and subsequently drying the hydrated sarsasapogenin crystals, preferably at a temperature below about 80°C, more preferably below about 70°C, more preferably below about 60°C, to form relatively pure substantially non-solvated non-hydrated crystalline sarsasapogenin.

The formation of crystalline form C sarsasapogenin is accomplished by any suitable means. In a preferred embodiment, the form C is obtained by dissolving relatively impure sarsasapogenin in an organic solvent, preferably in the absence of water, crystallising sarsasapogenin from the said solution, separating the crystals from at least the major part of the supernatant solvent, and slurrying the crystals in water to obtain form C. The sequential crystallisation from organic solvent, separation and wet slurrying is found to provide better purification than other methods of preparing form C sarsasapogenin described herein.

The organic solvent may conveniently be acetone, and the separation of the crystals from the supernatant may suitably be by filtration. The reslurrying of the separated crystals may suitably be achieved by adding the damp solid filtration product to hot water or hot aqueous solvent, suitably at about 40-60°C, and reslurrying for about 2-8 hours, to furnish form C, which is then suitably separated from the liquid (e.g. by filtration) and dried, suitably at about 50°C, to afford form B.

We have also found that the known non-hydrated non-solvated crystalline form B sarsasapogenin may be readily obtained in high purity and in a shorter process than that described above, by recrystallisation of any crystalline or amorphous form of sarsasapogenin (including hydrated or solvated forms) from a mixed alkane/ketone solvent. This observation has enabled us to develop an additional novel, practical, process for the large scale purification of sarsasapogenin.

Therefore, as also stated above, the present invention provides a method for purification of sarsasapogenin, particularly but not exclusively on a commercial manufacturing scale, which comprises dissolving sarsasapogenin (including any solvated, hydrated, crystalline or amorphous form thereof) in a mixed alkane/ketone solvent, preferably in the absence or substantial absence of water, and precipitating sarsasapogenin from the resulting solution as relatively pure substantially non-solvated non-hydrated crystalline sarsasapogenin.

The crystallisation may be conducted at any suitable temperature. Typically, the precipitation can be achieved at or somewhat below room temperature.

The direct crystallisation from an alkane/ketone solvent mixture is found to provide a very useful level of purification.

The alkane and the ketone used in the mixed alkane/ketone solvent will be any alkane and ketone which has acceptable physical properties at the desired operating temperature. The alkane may be straight or branched or cyclic or a mixture of straight, branched and/or cyclic alkanes may be used. The ketone may be a dialkyl ketone, which may be symmetrical or asymmetrical or a mixture of any two or more dialkyl ketones. The alkyl portions of the or each ketone may, independently of each other, be straight or branched or cyclic. Heptane and acetone are particularly mentioned as alkane and ketone respectively. The mixing ratio of the alkane and the ketone in the solvent may be varied between wide limits, and it is a simple matter for one of ordinary skill in this art to select a suitable ratio. The volume ratio of 3:1 heptane:acetone may be mentioned as a suitable example.

### Derivatives of sarsasapogenin

The term "derivatives" used herein refers particularly to the compounds defined and described in the prior art patent documents acknowledged above in relation to the known biological activities of sarsasapogenin (US Patent No. 4680289; WO-A-99/48507; WO-A-01/23406; WO-A-01/49703; WO-A-02/079221; and WO-A-03/082893).

Such derivatives include pharmaceutically acceptable pro-drugs of sarsasapogenin and pharmaceutically acceptable salts thereof.

Pro-drugs of sarsasapogenin may especially include 3-position carboxylate esters such as the cathylate (ethoxycarbonyloxy), acetate, succinate, propionate, butyrate, isobutyrate, valerate, isovalerate, caproate, isocaproate, diethylacetate, octanoate, decanoate, laurate, myristate, palmitate, stearate, benzoate, phenylacetate, phenylpropionate, cinnamate, p-nitrobenzoyloxy, 3,5-dinitrobenzoyloxy, p-chlorobenzoyloxy, 2,4-dichlorobenzoyloxy, p-bromobenzoyloxy, m-bromobenzoyloxy, p-methoxy-benzoyloxy, phthalyl, glycinate, alaninate, valinate, phenylalaninate, isoleucinate, methioninate, argininate, aspartate, cysteinate, glutaminate, histidinate, lysinate, prolinate, serinate, threoninate, tryptophanate, tyrosinate, fumarate and maleate esters.

"Pharmaceutically acceptable salts" means the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. See, for example S. M. Berge et al., Pharmaceutical Salts, J. Pharm. Sci., 66: pp.1-19 (1977) which is incorporated herein by reference. Base addition salts can also be prepared by separately reacting the purified compound in its acid form with a suitable organic or inorganic base and isolating the salt thus formed. Base addition salts include pharmaceutically acceptable metal and amine salts. Examples of suitable acid addition salts are those formed with acids selected from hydrochloric, sulphuric, phosphoric and nitric acids. Examples of suitable base addition salts are those formed with bases selected from sodium hydroxide, potassium hydroxide and ammonium hydroxide.

### Medicaments, Foodstuffs, Food Supplements, Beverages and Beverage Supplements

According to the invention, a composition may comprise a material as described above in admixture with one or more further component selected from: one or more other materials as described above, another form of sarsasapogenin, any other biologically active material, and any biologically inactive material.

The composition may be prepared by a method comprising admixing a material as described above with one or more further component selected from: one or more other materials as described above, another form of sarsasapogenin, any other biologically active material, and any biologically inactive material.

According to the invention the material or the composition (e.g. the medicament, foodstuff, food supplement, beverage or beverage supplement) may be used for non-therapeutic enhancement of cognitive function (i.e. in mentally healthy individuals to improve mental agility and without medical supervision) or for the therapeutic treatment of a condition selected from: obesity and diabetes obesity syndromes; cognitive dysfunction and allied conditions; conditions characterised by a deficiency in membrane-bound receptor number or function in a tissue, organ, cell type or organelle; non-cognitive neurodegeneration; non-cognitive neuromuscular degeneration; motor-sensory neurodegeneration; and loss of receptor function in the absence of cognitive, neural or neuromuscular impairment.

Still further, the invention therefore provides a non-therapeutic method for enhancing cognitive function in a human or non-human animal (i.e. in mentally healthy individuals to improve mental agility and without medical supervision) or a therapeutic method of treatment of a human or non-human animal (e.g. a human) suffering from, or susceptible to, a condition selected from: obesity and diabetes obesity syndromes; cognitive dysfunction and allied conditions; conditions characterised by a deficiency in membrane-bound receptor number or function in a tissue, organ, cell type or organelle; non-cognitive neurodegeneration; non-cognitive neuromuscular degeneration; motor-sensory neurodegeneration; and loss of receptor function in the absence of cognitive, neural or neuromuscular impairment, which comprises administering (including self-administering) to the said human or non-human animal an effective amount of a material or composition as decribed above.

The active agent prepared according to the present invention may thus be formulated into any suitable composition form for administration to a human or non-human animal patient. The composition may consist of the active agent alone or may include the active agent and any suitable additional component, such as one or more pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms.

The composition may, for example, be a pharmaceutical composition (medicament), a foodstuff, food supplement, beverage or beverage supplement.

The terms "foodstuff", "food supplement", "beverage" and "beverage supplement" used herein have the normal meanings for those terms, and are not restricted to pharmaceutical preparations. The appropriate pharmaceutical or edible grade of ingredients will be used, according to the desired composition form.

For further details of suitable composition forms and dosages, and examples of conditions and disease states treatable using the materials and compositions of the present invention, please refer to US Patent No. 4680289; WO-A-99/48507; WO-A-01/23406; WO-A-01/49703; WO-A-02/07922; and WO-A-03/082893.

Specific conditions and disease states treatable using the materials and compositions of the present invention include, for example: Alzheimer's disease, senile dementia (including senile dementia of the Alzheimer's type), Lewi body dementia, Parkinson's disease, postencephalitic Parkinsonism, depression, schizophrenia, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, motor neurone diseases including amyotrophic lateral sclerosis (ALS), multiple sclerosis, postural hypotension, traumatic neurodegeneration e.g. following stroke or following an accident (for example, traumatic head injury or spinal cord injury), Batten's disease, Cockayne syndrome, Down syndrome, corticobasal ganglionic degeneration, multiple system atrophy, cerebral atrophy, olivopontocerebellar atrophy, dentatorubral atrophy, pallidoluysian atrophy, spinobulbar atrophy, optic neuritis, sclerosing pan-encephalitis (SSPE), attention deficit disorder, post-viral encephalitis, post-poliomyelitis syndrome, Fahr's syndrome, Joubert syndrome, Guillain-Barre syndrome, lissencephaly, Moyamoya disease, neuronal migration disorders, autism, autistic syndrome, polyglutamine disease, Niemann-Pick disease, progressive multifocal leukoencephalopathy, pseudotumor cerebri, Refsum disease, Zellweger syndrome, supranuclear palsy, Friedreich's ataxia, spinocerebellar ataxia type 2, Rhett syndrome, Shy-Drager syndrome, tuberous sclerosis, Pick's disease, chronic fatigue syndrome, neuropathies including hereditary neuropathy, diabetic neuropathy and mitotic neuropathy, prion-based neurodegeneration, including Creutzfeldt-Jakob disease (CJD), variant CJD, new variant CJD, bovine spongiform encephalopathy (BSE), GSS, FFI, kuru and Alper's syndrome, Joseph's disease, acute disseminated encephalomyelitis, arachnoiditis, vascular lesions of the central nervous system, loss of extremity neuronal function, Charcot-Marie-Tooth disease, susceptibility to heart failure, asthma, and macular degeneration.

### Brief Description of the Drawings

In the accompanying drawings:
Figure 1 shows an X-ray powder diffraction (XRPD) pattern (λ = 1.5406 Angstroms) obtained from a sample of commercially available sarsasapogenin in crystalline form B (prior art);
Figure 2 shows a differential scanning calorimetry (DSC) trace obtained from a sample of commercially available sarsasapogenin in crystalline form B (prior art);
Figure 3 shows a thermogravimetric analysis (TGA) trace obtained from a sample of commercially available sarsasapogenin in crystalline form B (prior art);
Figure 4 shows an X-ray powder diffraction (XRPD) pattern (λ = 1.5406 Angstroms) obtained from a sample of sarsasapogenin in crystalline form A;
Figure 5 shows a differential scanning calorimetry (DSC) trace obtained from a sample of sarsasapogenin in crystalline form A;
Figure 6 shows a thermogravimetric analysis (TGA) trace obtained from a sample of sarsasapogenin in crystalline form A;
Figure 7 shows an X-ray powder diffraction (XRPD) pattern (λ = 1.5406 Angstroms) obtained from a sample of sarsasapogenin in crystalline form C;
Figure 8 shows a differential scanning calorimetry (DSC) trace obtained from a sample of sarsasapogenin in crystalline form C;
Figure 9 shows a thermogravimetric analysis (TGA) trace obtained from a sample of sarsasapogenin in crystalline form C;
Figure 10 shows an X-ray powder diffraction (XRPD) pattern (λ = 1.5406 Angstroms) obtained from a sample of sarsasapogenin in crystalline form D;
Figure 11 shows a differential scanning calorimetry (DSC) trace obtained from a sample of sarsasapogenin in crystalline form D;
Figure 12 shows a thermogravimetric analysis (TGA) trace obtained from a sample of sarsasapogenin in crystalline form D;
Figure 13 shows an X-ray powder diffraction (XRPD) pattern (λ = 1.5406 Angstroms) obtained from a sample of sarsasapogenin in crystalline form E;
Figure 14 shows a differential scanning calorimetry (DSC) trace obtained from a sample of sarsasapogenin in crystalline form E;
Figure 15 shows a thermogravimetric analysis (TGA) trace obtained from a sample of sarsasapogenin in crystalline form E;
Figure 16 shows an X-ray powder diffraction (XRPD) pattern (λ = 1.5406 Angstroms) obtained from a sample of sarsasapogenin in crystalline form F;
Figure 17 shows a differential scanning calorimetry (DSC) trace obtained from a sample of sarsasapogenin in crystalline form F;
Figure 18 shows a thermogravimetric analysis (TGA) trace obtained from a sample of sarsasapogenin in crystalline form F;
Figure 19 shows an X-ray powder diffraction (XRPD) pattern (λ = 1.5406 Angstroms) obtained from a sample of sarsasapogenin in crystalline form G;
Figure 20 shows a differential scanning calorimetry (DSC) trace obtained from a sample of sarsasapogenin in crystalline form G;
Figure 21 shows a thermogravimetric analysis (TGA) trace obtained from a sample of sarsasapogenin in crystalline form G;
Figure 22 shows an X-ray powder diffraction (XRPD) pattern (λ = 1.5406 Angstroms) obtained from a sample of sarsasapogenin in crystalline form H;
Figure 23 shows a differential scanning calorimetry (DSC) trace obtained from a sample of sarsasapogenin in crystalline form H;
Figure 24 shows a thermogravimetric analysis (TGA) trace obtained from a sample of sarsasapogenin in crystalline form H;
Figure 25 shows an X-ray powder diffraction (XRPD) pattern (λ = 1.5406 Angstroms) obtained from a sample of sarsasapogenin in crystalline form I;
Figure 26 shows a differential scanning calorimetry (DSC) trace obtained from a sample of sarsasapogenin in crystalline form I;
Figure 27 shows a thermogravimetric analysis (TGA) trace obtained from a sample of sarsasapogenin in crystalline form I;
Figure 28 shows an X-ray powder diffraction (XRPD) pattern (λ = 1.5406 Angstroms) obtained from a sample of sarsasapogenin in crystalline form J;
Figure 29 shows a differential scanning calorimetry (DSC) trace obtained from a sample of sarsasapogenin in crystalline form J;
Figure 30 shows a thermogravimetric analysis (TGA) trace obtained from a sample of sarsasapogenin in crystalline form J;
Figure 31 shows an X-ray powder diffraction (XRPD) pattern (λ = 1.5406 Angstroms) obtained from a sample of sarsasapogenin in crystalline form K;
Figure 32 shows a differential scanning calorimetry (DSC) trace obtained from a sample of sarsasapogenin in crystalline form K;
Figure 33 shows an X-ray powder diffraction (XRPD) pattern (λ = 1.5406 Angstroms) obtained from a sample of sarsasapogenin 3-methyl-butanol solvate in crystalline form L;
Figure 34 shows an X-ray powder diffraction (XRPD) pattern (λ = 1.5406 Angstroms) obtained from a sample of sarsasapogenin methanol THF solvate in crystalline form M;
Figure 35 shows an X-ray powder diffraction (XRPD) pattern (λ = 1.5406 Angstroms) obtained from a sample of sarsasapogenin methanol water solvate in crystalline form N;
Figure 36 shows an X-ray powder diffraction (XRPD) pattern (λ = 1.5406 Angstroms) obtained from a sample of sarsasapogenin diethyleneglycol monomethyl ether solvate in crystalline form O;
Figure 37 shows an X-ray powder diffraction (XRPD) pattern (λ = 1.5406 Angstroms) obtained from a sample of sarsasapogenin aminoethanol solvate in crystalline form P; and
Figure 38 shows an X-ray powder diffraction (XRPD) pattern (λ = 1.5406 Angstroms) obtained from a sample of sarsasapogenin phenylethylamine solvate in crystalline form Q.

### Examples and Detailed Description of the Drawings

The following non-limiting Examples are provided as further illustration of the present invention, but without limitation, and are discussed with reference to the drawings.

### Test Equipment

The accompanying figures were obtained on the following equipment:

### XRPD

All XRPD patterns in this application were obtained using a Bruker C2 diffractometer equipped with a XYZ stage, a laser video microscope and a HiStar area detector. Typical collection times were 200s. The sealed copper tube (Cu Kα radiation: 1.5406 Angstroms) voltage and current were set at 40 kV and 40 mA respectively. The X-ray optics on the C2 apparatus consisted of a single Gobel mirror coupled with a pinhole collimator of 0.3 mm diameter. The beam divergence (effective size of the X-ray spot) yielded a value of approximately 4 mm.

### DSC

All DSC data in this application were collected on a TA Instruments Q1000 apparatus equipped with autosampler and carousel capable of holding 50 sample pans. The energy and temperature calibration standard was indium. Samples were heated at a rate of 10 degrees C per minute between 20 and 210°C under N₂ purge. Between 1 and 5 mg of each sample was used. Unsolvated samples were examined in a non-hermetically sealed aluminium pan. Solvated samples were examined using hermetically sealed pans to prevent damage to the DSC apparatus.

### TGA

All TGA data in this application were collected on a TA Instruments Q500 thermogravimetric analyser. The instrument was calibrated with nickel/alumel according to the manufacturer's instructions and a scan rate of 10 °C per minute was employed along with a nitrogen purge. Typically, the sample size was 10-20 mg and each material was loaded into aluminium DSC pans mounted inside platinum crucibles. Both holders were tared prior to any recordings.

### Starting Materials

Samples of commercially available sarsasapogenin were purchased from Steraloids Inc.

The samples were analysed and defined as form B by our nomenclature.

### Example 1

### Crystalline Form A (acetone hemi-solvate)

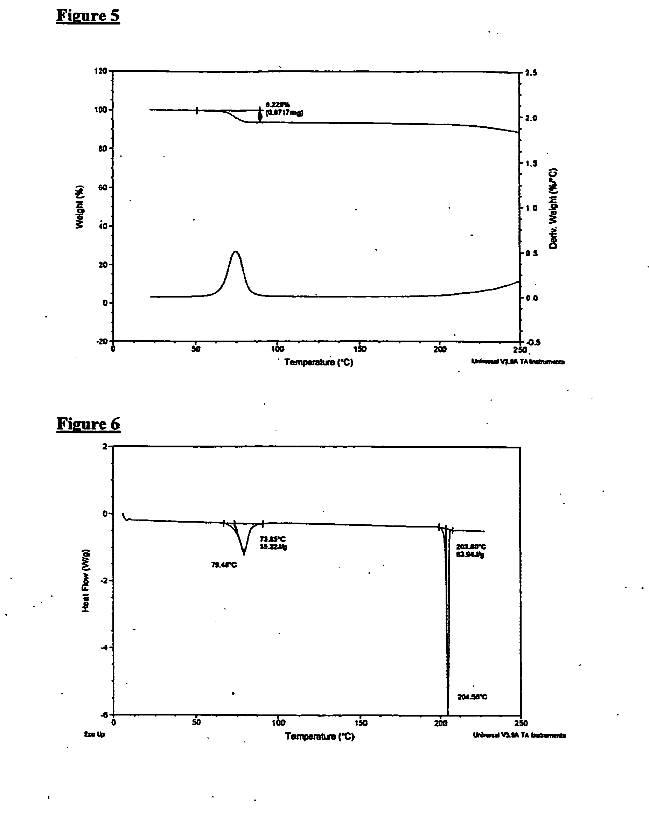

Sarsasapogenin (50 g) was dissolved in acetone (2500 ml) by heating to reflux with stirring. After 30 minutes the solution allowed to cool. At 25°C an ice bath was applied and the solution was cooled to 10°C. The solid was filtered and washed with acetone (100 ml) to yield white plate-like crystals. The solid was dried in a vacuum oven at 50°C for 16 hours to yield 37.85 g of material, which was characterised by XRPD as form A under our nomenclature. The XRPD, DSC, TGA are shown in Figures 4 to 6 of the accompanying drawings.

### Examples 2 and 3

### Crystalline Form B

### Example 2

A sample of the above batch (2.32 g) was dried in vacuum oven at 90°C for 3 days to afford form B. The XRPD trace corresponded to that shown in Figure 1 of the accompanying drawings.

### Example 3

Sarsasapogenin (240 g) was charged to a 20 litre flask followed by acetone (14.4 litres) and the mixture heated to reflux with stirring to dissolve the solid. The resultant hazy solution was heated at reflux for about 2.5 hours and filtered hot through a GFF/54 filter paper. The mixture was heated to reflux to redissolve the solids and then allowed to slowly cool with stirring. The mixture was further cooled with an ice/water bath to +2°C and the solids harvested by filtration on a GFF/54 filter paper (about 30 minutes). The solid was washed with cold acetone (+5°C; 360 ml).

The crude solid was reslurried in demineralised water (3200 ml) at 60-66°C for about 2 hours. The product was filtered hot (about 60 seconds) and washed with demineralised water (20°C; 2 x 480 ml). The solid was dried in a vacuum oven at 70°C to afford form B (193.7 g, 81% yield). The sample analysed for 0.3% water by Karl Fischer analysis.

The XRPD, DSC and TGA are shown in Figures 1 to 3 of the accompanying drawings.

### Examples 4, 5 and 6

### Crystalline Form C (hydrate)

### Example 4

Sarsasapogenin (99.4 g) was absorbed onto silica (about 200 g) using dichloromethane (2 litres). The material was packed at the top of an open column contain silica (2 kg). Chromatography using ethyl acetate/dichloromethane (1:19) as eluent afforded a product (89.1 g) which was recrystallised from acetone (3 litres) to afford an acetone wet cake (81.2 g). This material was further recrystallised from acetone (2.75 litres) to afford a solid that was dried at 50°C overnight. ¹H NMR spectroscopy indicated the presence of about 0.5% acetone, so further drying at 80°C was undertaken overnight to afford sarsasapogenin (71.2 g) as a white powder, which was characterised by XRPD as form C under our nomenclature, melting point 203.7-206.5°C. The elemental analysis result of C 76.69%; H 10.51% is consistent with the theoretical value for C₂₇H₄₄O₃.0.33H₂O. Water content by Karl-Fischer analysis was 1.41% w/w.

The XRPD, DSC and TGA are shown in Figures 7 to 9 of the accompanying drawings.

### Example 5

Exposure of the acetone solvate to a humidity of 50% or greater led to the formation of form C.

### Example 6

Sarsasapogenin (100 mg) was suspended in tetrahydrofuran (1 ml) and water (1 ml). The mixture was heated at 70°C overnight and allowed to cool over a 12 hour period. The precipitate was filtered, dried and shown to be form C.

### Examples 7, 8 and 9

### Crystalline Form D (ethanol solvate)

### Example 7

Sarsasapogenin (3.28 g) was dissolved in ethanol (65 ml) by heating to reflux. The mixture was allowed to cool and stirred overnight at ambient temperature. The mixture was further cooled in an ice bath for 1-2 hours and filtered. The solid was washed with cold ethanol (10 ml) and dried in a vacuum oven to afford form D (3.0 g). The sample analysed for 1% water by Karl Fischer analysis

The XRPD, DSC and TGA are shown in Figures 10 to 12 of the accompanying drawings.

### Example 8

Sarsasapogenin (3.48 g) was dissolved in ethanol (65 ml) by heating to reflux. The mixture was allowed to cool and stirred overnight at ambient temperature. The mixture was further cooled in an ice bath for 1-2 hours and filtered. The solid was washed with water and dried in a vacuum oven to afford form D (3.2 g). The sample analysed for 0.8% water by Karl Fischer analysis.

### Example 9

Sarsasapogenin (3.57 g) was dissolved in ethanol (70 ml) by heating to reflux. Water (9 ml) was added, causing some precipitation. Additional ethanol (30 ml) was added to effect dissolution. The mixture was allowed to cool and stirred overnight and for 2 days at ambient temperature. The mixture was filtered, the solid washed with water and dried in a vacuum oven to afford form D (3.18 g). The sample analysed for 0.8% water by Karl Fischer analysis.

### Example 10

### Crystalline Form H (not hydrated or solvated)

Heating a sample of the ethanol solvate (form D) at 130°C (i.e. above the desolvation temperature) in a vacuum oven resulted in "form H".

The XRPD, DSC and TGA are shown in Figures 22 to 24 of the accompanying drawings.

### Example 11

### Crystalline Form G (n-butanol solvate)

Sarsasapogenin (100 mg) was suspended in n-butanol (2 ml). The mixture was heated at 70°C for 1 hour and allowed to cool over a 3 hour period. The precipitate was filtered, dried and shown to be the n-butanol solvate.

The XRPD, DSC and TGA traces are shown in Figures 19 to 21 of the accompanying drawings.

### Example 12

### Crystalline Form E (n-propanol solvate)

Sarsasapogenin (100 mg) was suspended in n-propanol (2 ml). The mixture was heated at 70°C for 1 hour and allowed to cool over a 3 hour period. The precipitate was filtered, dried and shown to be the n-propanol solvate.

The XRPD, DSC and TGA traces are shown in Figures 13 to 15 of the accompanying drawings.

### Example 13

### Crystalline Form F (iso-propanol solvate)

Sarsasapogenin (100 mg) was suspended in *iso*-propanol (2 ml). The mixture was heated at 70°C for 1 hour and allowed to cool over a 3 hour period. The precipitate was filtered, dried and shown to be the *iso*-propanol solvate.

The XRPD, DSC and TGA traces are shown in Figures 16 to 18 of the accompanying drawings.

### Example 14

### Form I (0.3-hydrate)

Sarsasapogenin (form H; 50 mg) was stored at 40°C and 75% relative humidity for 18 days to furnish form 1.

The XRPD, DSC and TGA traces are shown in Figures 25 to 27 of the accompanying drawings.

### Example 15

### Form J (tert-butyl methyl ether solvate)

Sarsasapogenin (300 mg) was stirred in tert-butyl methyl ether at 50°C for 5 min. The mixture was allowed to cool and stirred overnight. The solid was harvested by filtration, washed with tert-butyl methyl ether (4 ml) and air dired to furnish form J.

The XRPD, DSC and TGA traces are shown in Figures 28 to 30 of the accompanying drawings.

### Example 16

### Form K (hemi-methanol solvate)

Sarsasapogenin was recrystallised from methanol to furnish form K.

The XRPD and DSC traces are shown in Figures 31 and 32 of the accompanying drawings.

### Example 17

### Form L (3-methyl-1-butanol solvate)

Sarsasapogenin was recrystallised from 3-methyl-1-butanol to furnish form L.

The XRPD trace is shown in Figure 33 of the accompanying drawings and the unit cell dimensions, angles and space group are shown in Table 1 above.

### Example 18

### Form M (mono-methanol, hemi-THF solvate)

Sarsasapogenin was recrystallised from a mixture of methanol and tetrahydrofuran (THF) containing one mole equivalent of raffinose, to furnish form M.

The XRPD trace is shown in Figure 34 of the accompanying drawings and the unit cell dimensions, angles and space group are shown in Table 1 above.

### Example 19

### Form N mono-methanol, mono-water solvate)

Sarsasapogenin was recrystallised from a mixture of methanol and tetrahydrofuran (THF) containing one mole equivalent of sucrose, to furnish form N.

The XRPD trace is shown in Figure 35 of the accompanying drawings and the unit cell dimensions, angles and space group are shown in Table 1 above.

### Example 20

### Form O (diethyleneglycol monomethyl ether solvate)

Sarsasapogenin was recrystallised from diethyleneglycol monomethyl ether to furnish form O.

The XRPD trace is shown in Figure 36 of the accompanying drawings and the unit cell dimensions, angles and space group are shown in Table 1 above.

### Example 21

### Form P (aminoethanol solvate)

Sarsasapogenin was recrystallised from aminoethanol to furnish form P.

The XRPD trace is shown in Figure 37 of the accompanying drawings and the unit cell dimensions, angles and space group are shown in Table 1 above.

### Example 22

### Form Q (phenylethylamine solvate)

Sarsasapogenin was recrystallised from phenylethylamine to furnish form Q.

The XRPD trace is shown in Figure 38 of the accompanying drawings and the unit cell dimensions, angles and space group are shown in Table 1 above.

The foregoing broadly describes the present invention, without limitation. Variations and modifications as will be readily apparent to those of ordinary skill in this art are intended to be included in the scope of this application and any resultant patent.

## Claims

1. A crystalline hydrate of sarsasapogenin that contains between 0.25 and 0.75 moles of water per mole of sarsasapogenin.

2. The crystalline hydrate of claim 1 that contains between 0.3 and 0.5 moles of water per mole of sarsasapogenin.

3. The crystalline hydrate of claim 1 that is a sarsasapogenin hemihydrate.

4. The crystalline hydrate of claims 1 to 3 in at least about 90% by weight pure form.

5. The crystalline hydrate of claims 1-4 prepared by crystallising sarsapogenin from organic solvent, separating the crystals from at least a major part of the solvent and slurrying the crystals in water.

6. The crystalline hydrate of claim 5 wherein the slurrying is carried out in hot water or hot aqueous solvent.

7. The crystalline hydrate of claim 6 wherein the hot water or hot aqueous solvent is at a temperature of 40-60 °C.

8. A process for obtaining pharmaceutical or edible grade sarsasapogenin or a derivative thereof, wherein at least one step in the process includes preparing a material according to any one of claims 1-7.

9. A method of adjusting the hydration level of sarsasapogenin comprising crystallising sarsapogenin from organic solvent, separating the crystals from at least a major part of the solvent and slurrying the crystals in water.

10. The method according to claim 10 wherein the adjusted form of sarsapogenin comprises a material according to any one of claims 1-7.

11. A method of preparing a prodrug of sarsapogenin which comprises esterifying a sarsasapogening material according to any one of claims 1-7.

12. A prodrug of sarsasapogenin obtained by a method according to claim 11.

13. Use of a material according to claims 1-7 in the manufacture of a medicament, food supplement or beverage supplement.

14. Use according to claim 13 wherein the medicament, food supplement or beverage supplement is for the enhancement of cognitive function or for the treatment of a condition selected from: obesity and diabetes obesity syndromes; cognitive dysfunction and allied conditions; conditions **characterised by** a deficiency in membrane-bound receptor number or function; non-cognitive neurodegeneration; non-cognitive neuromuscular degeneration; motor-sensory neurodegeneration; and loss of receptor function in the absence of cognitive, neural or neuromuscular impairment.
